# EUROPEAN PATENT SPECIFICATION

(11) **EP 1 183 393 B1**
(45) Date of publication and mention of the grant of the patent: **21.06.2006**
(21) Application number: 01911285.3
(22) Date of filing: 15.03.2001
(51) Int. Cl.: C12Q 1/68

(54) **PCR-BASED IDENTIFICATION OF EIMERIA SPECIES AND STRAINS**
IDENTIFIZIERUNG VON EIMERIA SPEZIES UND STÄMMEN AUF PCR-BASIS
IDENTIFICATION PAR PCR D'ESPECES ET DE SOUCHES D'EIMERIA

(30) Priority: 15.03.2000 AU PQ622900
(43) Date of publication of application: 06.03.2002
(73) Proprietor: Eimeria Pty Ltd, Melbourne VIC 3000 (AU)
(72) Inventor: GASSER, Robin, Beat, Werribee, VIC 3030 (AU); WOODS, Wayne, Geoffery, Sunbury, VIC 3429 (AU); RICHARDS, David, Grant, Frankston North, VIC 3199 (AU); WHITHEAR, Kevin, George, Port Melbourne, VIC 3207 (AU)
(74) Representative: Flaccus, Rolf-Dieter
(86) International application number: PCT/AU2001/000291
(87) International publication number: WO 2001/068909

(56) References cited:
- EP-A- 0 516 381
- AU-B2- 656 378
- BARTA JOHN R: "Investigating phylogenetic relationships within the Apicomplexa using sequence data: The search for homology." METHODS (ORLANDO), vol. 13, no. 2, October 1997 (1997-10), pages 81-88, XP002233985 ISSN: 1046-2023 -& DATABASE GENBANK [Online] 16 December 1997 (1997-12-16) BARTA J.R. ET AL.: "Eimeria maxima strain Guelph 18S ribosomal RNA gene, partial sequence; internal transcribed spacer 1, 5.8S ribosomal RNA gene and internal transcribed spacer 2, complete sequence; and 28S ribosomal RNA gene, partial sequence." retrieved from GENBANK Database accession no. AF027726 XP002233988 -& DATABASE GENBANK [Online] 16 December 1997 (1997-12-16) BARTA J.R. ET AL.: "Eimeria maxima strain Florida 18S ribosomal RNA gene, partial sequence; internal transcribed spacer 1, 5.8S ribosomal RNA gene and internal transcribed spacer 2, complete sequence; and 28S ribosomal RNA gene, partial s
- BARTA J R ET AL: "Analysis of infraspecific variation among five strains of Eimeria maxima from North America." INTERNATIONAL JOURNAL FOR PARASITOLOGY, vol. 28, no. 3, March 1998 (1998-03), pages 485-492, XP002233986 ISSN: 0020-7519
- TURENNE CHRISTINE Y ET AL: "Rapid identification of fungi by using the ITS2 genetic region and an automated fluorescent capillary electrophoresis system." JOURNAL OF CLINICAL MICROBIOLOGY, vol. 37, no. 6, June 1999 (1999-06), pages 1846-1851, XP002233984 ISSN: 0095-1137
- GASSER ROBIN B: "Mutation scanning methods for the analysis of parasite genes." INTERNATIONAL JOURNAL FOR PARASITOLOGY, vol. 27, no. 12, December 1997 (1997-12), pages 1449-1463, XP002233987 ISSN: 0020-7519
- TSUJI NAOTOSHI ET AL: "Discrimination of eight chicken Eimeria species using the two-step polymerase chain reaction." JOURNAL OF PARASITOLOGY, vol. 83, no. 5, 1997, pages 966-970, XP009006980 ISSN: 0022-3395
- DATABASE GENBANK [Online] 30 October 1997 (1997-10-30) LITTLEBURY P.L. AND TOMLEY F.M.: "Eimeria tenella small subunit ribosomal RNA, 5.8S ribosomal RNA, and large subunit ribosomal RNA genes, and internal transcribed spacer 1 and 2, complete sequence." retrieved from GENBANK Database accession no. AF026388 XP002233993
- DATABASE GENBANK [Online] 7 May 1997 (1997-05-07) PROFOUS-JUCHELKA H.: "Eimeria mitis small subunit ribosomal RNA gene, complete sequence." retrieved from GENBANK Database accession no. U67118 XP002233994
- DATABASE GENBANK [Online] 7 May 1997 (1997-05-07) PROFOUS-JUCHELKA H.: "Eimeria mitis small subunit ribosomal RNA gene, complete sequence." retrieved from GENBANK Database accession no. U67115 XP002233995
- DATABASE GENBANK [Online] 7 May 1997 (1997-05-07) PROFOUS-JUCHELKA H.: "Eimeria tenella small subunit ribosomal RNA gene, complete sequence." retrieved from GENBANK Database accession no. U67121 XP002233996
- DATABASE GENBANK [Online] 22 February 1996 (1996-02-22) RELMAN D.A.: "Eimeria mitis small subunit ribosomal RNA gene, complete sequence." retrieved from GENBANK Database accession no. U40262 XP002233997
- WOODS W.G. ET AL.: 'Single-strand restriction fragment length polymorphism analysis of the second internal transcribed spacer (ribosomal DNA) for six species of eimeria from chickens in Australia' INT. J. PARASITOL. vol. 30, 2000, pages 1019 - 1023, XP001068271
- SCHNITZLER B.E. ET AL.: 'PCR identification of chicken eimeria: a simplified read-out' AVIAN PATHOL. vol. 28, 1999, pages 89 - 93, XP001068258
- STUCKI U. ET AL.: 'Eimeria tenella: Characterization of a 5S ribosomal RNA repeats unit and its use as a species-specific probe' EXP. PARASITOL. vol. 76, 1993, pages 68 - 75, XP001068261
- DATABASE GENBANK [Online] 05 November 1997 'Eimeria tenella small subunit ribosomal RNA, 5.8S ribosomal RNA and large subunit ribosomal RNA gene and internal transcribed spacer 1 and 2', XP002950690 Database accession no. AF026388
- DATABASE GENBANK [Online] 29 December 1998 'Eimeria maxima internal transcribed spacer 1', XP002950691 Database accession no. AF065094
- DATABASE GENBANK [Online] 04 December 1998 'Eimeria maxima strain USDA 68 18S ribosomal RNA gene, partial sequence; internal transcribed spacer 1, 5.8S ribosomal RNA gene and internal transcribed spacer 2, complete sequence and 28S ribosomal RNA gene', XP002950692 Database accession no. AF027722

### SEQUENCE LISTING

<110> GASSER, Robin B.
   WOODS, Wayne G.
   RICHARDS, David G.
   WHITHEAR, Kevin G.
   THE UNIVERSITY OF MELBOURNE
<120> PCR-BASED IDENTIFICATION OF EIMERIA SPECIES AND STRAINS
<130> 7464793/PAS/SAR
<140>
   <141>
<160> 30
<170> PatentIn Ver. 2.1
<210> 1
   <211> 626
   <212> DNA
   <213> Eimeria tenella
<400> 1
   tctaaaggat gcaaaagtcg taacacggtt tccgtagtga acctgcggaa ggatcattca 60
   cacaattcgc acgcctggaa cgcgctgctg gttttacagg tttcaagcat tcgctttgcc 120
   tgggtggcca gcagcaggta gtcgtcggtg ttgttggaaa gaaaacttta gtccatcgca 180
   acccttgaat ctgtttttct ctgcaacggt ttttctactt tttaaaaatt gaaggaattt 240
   tttgctgctg caaggatata tcgcagtagt atgtacgtgg gcgatcgggg gggtggtggc 300
   gcatgcacgg gctcgcgtgg ggcctgtcgg tggcagcccc agcgcgccgg cgcagccccg 360
   tgatcgtcga tcgcgcacgt acgtggaggg gattatgaga ggagaagacg cgcacggggc 420
   tgtgtcgtat gcagagcgct cgcggctcgg gcgattgttc cgtgttgtgt gctctgctgc 480
   atgctggtgt gtgcgttctg tctctctctc tctccgttac atgctgcttg gaactttgct 540
   tcagcaagaa acctttgctc actaaggtga atcgaatcac ttttgttgat gagcagaagg 600
   aagaataggt agaaatcgga aaaaac                                      626
<210> 2
   <211> 512
   <212> DNA
   <213> Eimeria tenella
<400> 2
   cttaacaact cctactagta ggccatgctg ctgtctgtct ctgttccttg tggtcctgtg 60
   agggttcggc gatgctgccg acagaagtga gtgctttgct cgtttctgtt ttgtgtcgcg 120
   gaattttttc gggtcaccaa aggggaggta gaagcatgtt tggtttcatt tgagtgtcgt 180
   tgcattggtt ttgaaggaga tgcggcgtct ctcgaaattg ttgtcggcag cggtgctgtg 240
   tgtctgcaca gtgtgccgtt ttccatgcct gtgctttcta tagtgtcgtc gtatgctcct 300
   ttcattcgga aagagagaga tacggtggtt gtattttatg caacgttgtt tgtctcgttc 360
   tggacgaatg ttttgagcag ggctagggcg aggtataata gtgcatgggt atgcgacaac 420
   gtgaaacgac atatagtaca cggcaccatg gacgtgttgc atgcgtcgtt ttttttcggt 480
   attacacatg tatgtataga cctgaaatca gt                               512
<210> 3
   <211> 10
   <212> DNA
   <213> synthetic construct
<400> 3
   tctaaaggat 10
<210> 4
   <211> 4
   <212> DNA
   <213> synthetic construct
<400> 4
   cagc 4
<210> 5
   <211> 5
   <212> DNA
   <213> synthetic construct
<400> 5
   gtttt 5
<210> 6
   <211> 10
   <212> DNA
   <213> synthetic construct
<400> 6
   atgcgtgagc 10
<210> 7
   <211> 10
   <212> DNA
   <213> synthetic construct
<400> 7
   actgatttca 10
<210> 8
   <211> 10
   <212> DNA
   <213> synthetic construct
<400> 8
   tgatatgctt 10
<210> 9
   <211> 19
   <212> DNA
   <213> synthetic construct
<400> 9
   agttgcgtaa atagagccc 19
<210> 10
   <211> 19
   <212> DNA
   <213> synthetic construct
<400> 10
   aagacatcca ttgctgaaa 19
<210> 11
   <211> 18
   <212> DNA
   <213> synthetic construct
<400> 11
   aagttgcgta aaagagcc 18
<210> 12
   <211> 19
   <212> DNA
   <213> synthetic construct
<400> 12
   caagacatcc attgctgaa 19
<210> 13
   <211> 18
   <212> DNA
   <213> synthetic construct
<400> 13
   aagttgcgta aatagagc 18
<210> 14
   <211> 18
   <212> DNA
   <213> synthetic construct
<400> 14
   caagacatcc attgctga 18
<210> 15
   <211> 17
   <212> DNA
   <213> synthetic construct
<400> 15
   ttgcgtaaat agagccc 17
<210> 16
   <211> 17
   <212> DNA
   <213> synthetic construct
<400> 16
   gacatccatt gctgaaa 17
<210> 17
   <211> 17
   <212> DNA
   <213> synthetic construct
<400> 17
   cgtctgtttc agtgtct 17
<210> 18
   <211> 19
   <212> DNA
   <213> synthetic construct
<400> 18
   aattcagcgg gtaacctcg 19
<210> 19
   <211> 17
   <212> DNA
   <213> synthetic construct
<400> 19
   acgtctgttt cagtgtc 17
<210> 20
   <211> 19
   <212> DNA
   <213> synthetic construct
<400> 20
   aaattcagcg ggtaacctc 19
<210> 21
   <211> 17
   <212> DNA
   <213> synthetic construct
<400> 21
   acgtctgttt cagtgct 17
<210> 22
   <211> 18
   <212> DNA
   <213> synthetic construct
<400> 22
   aaattcagcg ggtaacct 18
<210> 23
   <211> 16
   <212> DNA
   <213> synthetic construct
<400> 23
   gtctgtttca gtgtct 16
<210> 24
   <211> 17
   <212> DNA
   <213> synthetic construct
<400> 24
   ttcagcgggt aacctcg 17
<210> 25
   <211> 20
   <212> DNA
   <213> synthetic construct
<400> 25
   aagttgcgta aatagagccc 20
<210> 26
   <211> 20
   <212> DNA
   <213> synthetic construct
<400> 26
   caagacatcc attgctgaaa 20
<210> 27
   <211> 18
   <212> DNA
   <213> synthetic construct
<400> 27
   acgtctgttt cagtgtct 18
<210> 28
   <211> 20
   <212> DNA
   <213> synthetic construct
<400> 28
   aaattcagcg ggtaacctcg 20
<210> 29
   <211> 19
   <212> DNA
   <213> synthetic construct
<400> 29
   acctggttga tcctgccag 19
<210> 30
   <211> 21
   <212> DNA
   <213> synthetic construct
<400> 30
   cttccgcagg ttcacctacg g 21

## Description

### FIELD OF THE INVENTION

The present invention relates to a PCR-based method of identifying various species of the genus *Eimeria* (commonly known as coccidia). More particularly, the present invention relates to a PCR-based method which is genus-specific, utilises novel PCR primers, and has the potential to identify species of *Eimeria* which may differ only by relatively minor sequence variation.

### BACKGROUND OF THE INVENTION

Bibliographic details of the publications referred to herein are collected at the end of the description.

Commonly used abbreviations in this text are: ITS, internal transcribed spacer; ITS-1, first internal transcribed spacer; ITS-2, second internal transcribed spacer; rDNA, ribosomal DNA; TBE, Tris-borate-EDTA; DPGE, denaturing polyacrylamide gel electrophoresis; SSCP, single-strand conformation polymorphism.

Coccidiosis is a disease of animals and birds caused by protozoan parasites (*Eimeria*) known as coccidia. This disease is of major economic importance to the poultry industry worldwide.

*Eimeria* species have complicated life cycles, details of which are well described. Briefly, when a sporulated (infective) coccidial oocyst is ingested, sporozoites are released to initiate asexual and sexual cycles that lead to the production of thousands of new oocysts, which are shed in the faeces of the host. These oocycts sporulate in the environment within days and are then infectious to naïve birds. A single sporulated oocyst may give rise to thousands of progeny. *Eimeria* species produce lesions in the gut by destruction of the epithelial cells in which they develop and multiply, and cause lesions in the intestinal wall.

The clinical signs of coccidiosis include diarrhoea, which may be mucoid or bloody, and dehydration. These symptoms are generally followed by ruffling of feathers, anaemia, listlessness, loss of weight, retraction of the head and neck and somnolence. Coccidiosis in laying hens is usually detected by a decrease in egg production. Infected birds cease to grow satisfactorily. With highly virulent strains of *Eimeria* morality in chickens is usually high.

Currently, seven species of *Eimeria* are recognised to infect chickens. These species differ considerably in their biology and pathogenicity (McDougald *et al,* 1998). Being able to accurately identify *Eimeria* species and "strains" has major implications for diagnosis and control as well as for studying their epidemiology and population biology.

Traditionally, species of *Eimeria* have been identified by a variety of methods. For example, morphological features and/or morphometry of their oocysts or sporocysts (size, shape, length and width), their patterns of development, the nature of the lesions they produce, their predilection site in the gut, or sporulation times and reproductive index. However, these criteria can be unreliable *(Eckert et al,* 1995). Biochemical, immunological and molecular methods (Andrews and Chilton, 1999; Gasser, 1997) can overcome such limitations but may themselves have other limitations.

Polymerase chain reaction (PCR) methods, using appropriate genetic markers, may be used as alternatives for identifying species of *Eimeria,* because of their ability to specifically amplify minute amounts of parasitic material (e.g. Stucki *et al*, 1993). However, such methods described to date are species-specific and may require the running of a number of different PCR reactions (using distinct pairs of species-specific oligonucleotide primers) in order to correctly identify a particular species of *Eimeria.*

For the molecular identification and classification of organisms, analysis of critical specific genomic regions is required. One such region in eukaryotes is within the ribosomal DNA (rDNA) of the nuclear genome. The rDNA of eukaryotes is a multigene family consisting of tandemly repeated units. Each unit comprises, an external transcribed spacer (ETS), the genes encoding the 18S, 5.8S and 28S rRNAs, separated by internal transcribed spacer regions (ITS-1 and ITS-2, respectively), and an intergenic spacer (IGS). Within this region, the sequences of ITS-1 and ITS-2 provide reliable genetic markers for the identification of organisms to the species level because intraspecific variation in these sequences is usually low compared with higher levels of interspecific difference.

It has been demonstrated that the ITS-1 and ITS-2 are useful genetic markers for the identification of species of *Eimeria* (Tsuji *et al,* 1997; Molloy *et al,* 1998; Schnitzler *et al,* 1999) or detection of population variation (Barta *et al,* 1998).

A PCR-based assay using species-specific primers in the ITS-1 for the typing of samples to species based on the detection of a product of a particular size on agarose gels has been developed (Schnitzler *et al,* 1999). However, such an assay does not allow sequence variation within a specific PCR product to be analysed and may further be disadvantaged by the fact that it is species-specific.

Further, Barta *et al,* 1998 have used a cloning/sequencing approach for the analysis of sequence variation both in the ITS-1 and ITS-2 sequences within *E. maxima.* However, this approach may have limitations, especially when large numbers of samples are require analysis as it is labour-intensive, time consuming and costly to perform. Moreover, it does not necessarily accurately resolve sequence variation among the different copies of rDNA, is species-specific, and may introduce artefacts into sequence data (Gasser, 1997). BARTA, J.R. ("Investigating phylogenetic relationships within the Apicomplexa using sequence data: The search for homology", 1997, Methods: A Companion to Methods in Enzymology 13, 81-88) describes the use of "universal" polymerase chain reaction primers which are complementary to the highly conserved regions of the 18S, 5.8 S and 28S rRNA genes, for PCR amplification of the 18S and 5.8S rRNA genes as well as the more highly variable ITS-1 and ITS-2 regions. Sequence data of the rRNA gene sequences were used for analyzing phylogenetic relationships within the Apicomplexa. ITS-1 and ITS-2 regions were found to contain sufficient primary sequence variation to be useful in discerning strain variation among five strains of Eimeria maxima.

EP-A-0516381 relates to a method for identifying individual species of Eimeria, by PCR amplification of genomic DNA and subsequent hybridization of the PCR products with Eimeria species-specific small subunit rRNA (ssrRNA) gene probes. Preferably, PCR primers are used which make it possible to selectively amplify each of the ssrRNA gene units or fragments thereof. Also disclosed are the nucleotide sequences for the ssrRNA genes of the various Eimeria species.

According to TURENNE, C. Y., et al. ("Rapid identification of fungi by using ITS2 genetic region and an automated fluorescent capillary electrophoresis system"; 1999, J. Clin. Microbiol., 37, 1846-1851), the ITS-2 region of various Candida yeasts did not reveal intra-specific variability among species for which more than one strain was tested. Therefore, the inter-specific sequence variability of the ITS-2 region may be used for the species-specific diagnosis of clinical fungal isolates. PCR with fungus-specific primers targeted toward conserved sequences of the 5.8S and 28S rRNA genes results in amplification of the species-specific ITS-2 regions which are variable in amplicon length. TSUJI, N. et al. ("Discrimination of eight chicken Eimeria species using the two-step polymerase chain reaction"; 1997, The Journal of Parasitology, 83, 966-970) describes a method for the discrimination of 8 Eimeria species. In a first step, the ssrRNA gene is amplified using conserved sequences for the Apicomplexa ssrRNA genes as primers. In the second step, the PCR products are subjected to a second PCR which is a RAPD-PCR (random amplification of polymorphic DNA), using arbitrary primers. Gel analysis of the RAPD-PCR products yields banding patterns which can be used to identify individual Eimeria species.

Due to the economic impact of coccidiosis on the poultry industry, for example, it is important that species of *Eimeria* are readily identifiable, such that rapid diagnosis of disease and treatment may occur. Further, sensitive and reliable identification of *Eimeria* is desirable for the study of the epidemiology of the diseases and for controlling the purity of laboratory lines of *Eimeria.* Accordingly, there is a need to develop an assay for the rapid identification of species of *Eimeria* which does not have the limitations of previously described assays.

More specifically, the problem to be solved by the present invention was to provide primers suitable for the amplification by PCR of specific regions of rDNA of *Eimeria* in a genus-specific manner, and to provide methods for detection and discrimination of *Eimeria* species based on PCR amplification of said regions of rDNA. This problem is solved by the present invention as follows:

In one aspect of the present invention there is provided an oligonucleotide comprising at least 15 consecutive bases of the DNA sequence designated SEQ ID NO: 25, or an annealing equivalent thereof, wherein said oligonucleotide is capable of specific hybridisation under PCR conditions to a target sequence being complementary to said at least 15 consecutive bases, and said oligonucleotide is adapted to prime the extension of DNA during PCR.

Preferably, the oligonucleotide comprises the sequence designated SEQ ID NO: 25. Alternatively, the oligonucleotide is chosen from the group consisting: SEQ ID NO: 9; SEQ ID NO: 11; SEQ ID NO: 13; and SEQ ID NO: 15.

In a second aspect of the present invention there is provided an oligonucleotide comprising at least 15 consecutive bases of the DNA sequence designated SEQ ID NO: 26, or an annealing equivalent thereof, wherein said oligonucleotide is capable of specific hybridisation under PCR conditions to a target sequence being complementary to said at least 15 consecutive bases, and said oligonucleotide is adapted to prime the extension of DNA during PCR.

Preferably, the oligonucleotide comprises the sequence designated SEQ ID NO: 26. Alternatively, the oligonucleotide is chosen from the group consisting: SEQ ID NO: 10; SEQ ID NO: 12; SEQ ID NO: 14; and SEQ ID NO: 16.

In a third aspect of the present invention there is provided an oligonucleotide comprising at least 15 consecutive bases of the DNA sequence designated SEQ ID NO: 27, or an annealing equivalent thereof, wherein said oligonucleotide is capable of specific hybridisation under PCR conditions to a target sequence being complementary to said at least 15 consecutive bases, and said oligonucleotide is adapted to prime the extension of DNA during PCR..

Preferably, the oligonucleotide comprises the sequence designated SEQ ID NO: 27. Alternatively, the oligonucleotide is chosen from the group consisting: SEQ ID NO: 17; SEQ ID NO: 19; SEQ ID NO: 21; and SEQ ID NO: 23.

In a fourth aspect of the present invention there is provided an oligonucleotide comprising at least 15 consecutive bases of the DNA sequence designated SEQ ID NO: 28, or an annealing equivalent thereof, wherein said oligonucleotide is capable of specific hybridisation under PCR conditions to a target sequence being complementary to said at least 15 consecutive bases, and said oligonucleotide is adapted to prime the extension of DNA during PCR.

Preferably, the oligonucleotide comprises the sequence designated SEQ ID NO: 28. Alternatively, the oligonucleotide is chosen from the group consisting: SEQ ID NO: 18; SEQ ID NO: 20; SEQ ID NO: 22; and SEQ ID NO: 24.

In a further aspect of the present invention there is provided a pair of PCR primers, one primer comprising at least 15 consecutive bases of the DNA sequence designated the SEQ ID NO: 25, or an annealing equivalent thereof, and a second primer comprising at least 15 consecutive bases of the DNA sequence designated SEQ ID NO: 26, or an annealing equivalent thereof.

Preferably, said pair of PCR primers comprises one primer comprising the DNA sequence designated SEQ ID NO: 25 and a second primer comprising the DNA sequence designated SEQ ID NO: 26. Alternatively, said pair of PCR primers comprises one of SEQ ID NO: 9; SEQ ID NO: 11; SEQ ID NO: 13; or SEQ ID NO: 15 and one of SEQ ID NO: 10; SEQ ID NO: 12; SEQ ID NO: 14; or SEQ ID NO: 16.

In a further aspect of the present invention there is provided a pair of PCR primers, one primer comprising at least 15 consecutive bases of the DNA sequence designated SEQ ID NO: 27, or an annealing equivalent thereof, and a second primer comprising at least 15 consecutive bases of the DNA sequence designated SEQ ID NO: 28, or an annealing equivalent thereof.

Preferably, said pair of PCR primers comprises a primer comprising the DNA sequence designated SEQ ID NO: 27 and a primer comprising the DNA sequence designated SEQ ID NO: 28. Alternatively, said pair of PCR primers comprises one of SEQ ID NO: 17; SEQ ID NO: 19; SEQ ID NO: 21; or SEQ ID NO: 23; and one of SEQ ID NO: 18; SEQ ID NO: 20 SEQ ID NO: 22; or SEQ ID NO: 24.

Preferably, said pairs of PCR primers as described herein are adapted to amplify specified regions of the rDNA of Eimeria in a genus-specific manner.

In yet a further aspect of the present invention there is provided a method of identifying *Eimeria* in a sample, said method comprising the steps:
providing a sample comprising genomic template DNA to be tested;
providing genomic DNA of one or more standards of known identity;
providing a pair of PCR primers selected from the group consisting of
   (i) primers comprising at least 15 consecutive bases of the DNA sequence designated SEQ ID NO: 25 and SEQ ID NO: 26 or annealing equivalents thereof; or
   (ii) primers comprising at least 15 consecutive bases of the DNA sequences designated SEQ ID NO: 27 and SEQ ID NO: 28 or annealing equivalents thereof;
   amplifying by means of PCR a region of template DNA using said primer pair to produce one or more PCR products from said sample, and said one or more standard of known identity;
   comparing said one or more PCR products derived from said sample against one or more PCR products of said one or more standard of known identity; and identifying the species of *Eimeria* present within the sample.

Preferably, said pair of PCR primers comprises the primers comprising the sequences designated SEQ ID NO: 25 and SEQ ID NO: 26. Alternatively, said pair of PCR primers comprises one of SEQ ID NO: 9; SEQ ID NO: 11; SEQ ID NO: 13; or SEQ ID NO: 15 and one of SEQ ID NO: 10; SEQ ID NO: 12; SEQ ID NO: 14; or SEQ ID NO: 16.

Alternatively, said pair of PCR primers comprises the primers comprising the sequences designated SEQ ID NO: 27 and SEQ ID NO: 28. Alternatively, said pair of PCR primers comprises one of SEQ ID NO: 17; SEQ ID NO: 19; SEQ ID NO: 21; or SEQ ID NO: 23 and one of SEQ ID NO: 18; SEQ ID NO: 20; SEQ ID NO: 22; or SEQ ID NO: 24.

Preferably, two PCRs are run per sample to be tested, wherein each PCR uses a different primer pair.

Alternatively, one PCR is run per sample to be tested and both primer pairs are provided within said one PCR.

Preferably said one or more PCR products derived from said sample are compared against said one or more PCR products of said one or more standard of known identity by means of gel electrophoresis.

Preferably, the gel electrophoresis is DPGE. Alternatively, the gel electrophoresis is SSCP. Alternatively, both DPGE and SSCP may be employed.

Preferably, gel electrophoresis is conducted in an automated system.

In a further aspect of the present invention there is provided the use of any one of the oligonucleotides as herein described or annealing equivalents thereof for the identification of species of *Eimeria.*

### FIGURES

These and other aspects of the present invention, which should be considered in all its novel aspects, will become apparent from the following description, which is given by way of example only, with reference to the accompanying figures, in which:

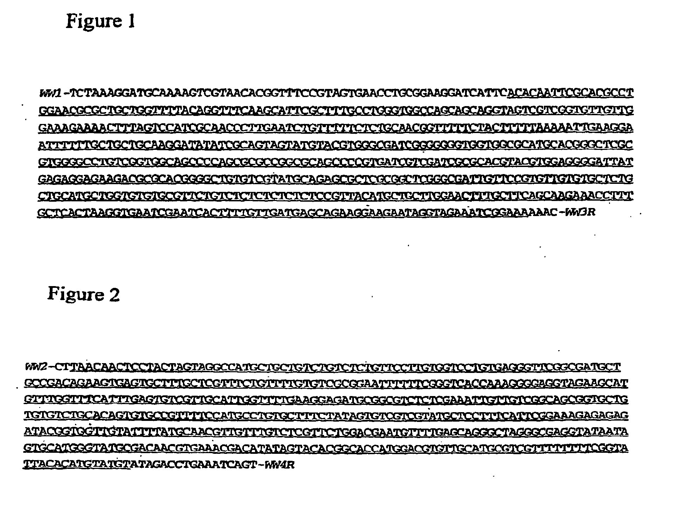

- **Figure 1**: Illustrates *E. tenella* ITS-1 sequence (SEQ ID NO: 1) amplified with primer pair WW1 and WW3r (shown in italics), the underlined sequence represents ITS-1 and plain text sequence the 3' end of 18s rDNA and 5' end of 5.8s rDNA;
- **Figure 2**: Illustrates *E. tenella* ITS-2 sequence (SEQ ID NO: 2) amplified with primer pair WW2 and WW4r (shown in italics), the underlined sequence represents ITS-2 and plain text sequence the 3' end of 5.8s rDNA and 5' end of 28s rDNA;

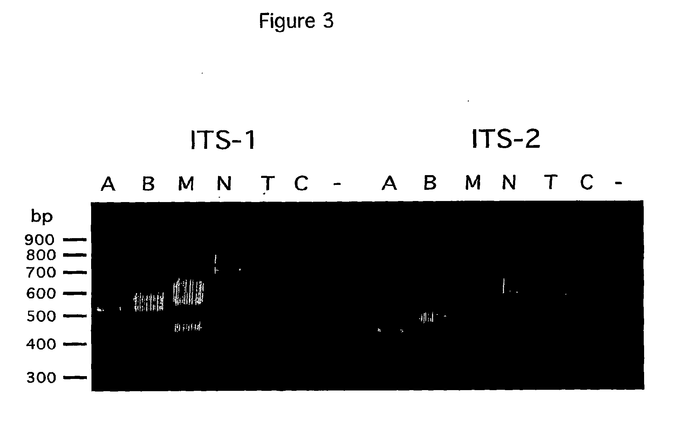

- **Figure 3**: Agarose gel showing ITS-1 or ITS-2 PCR products representing *E. acervulina, E. brunetti , E. maxima, E. necatrix, E. tenella* (A, B, M, N and T, respectively). Chicken DNA and no DNA controls (C and -, respectively);

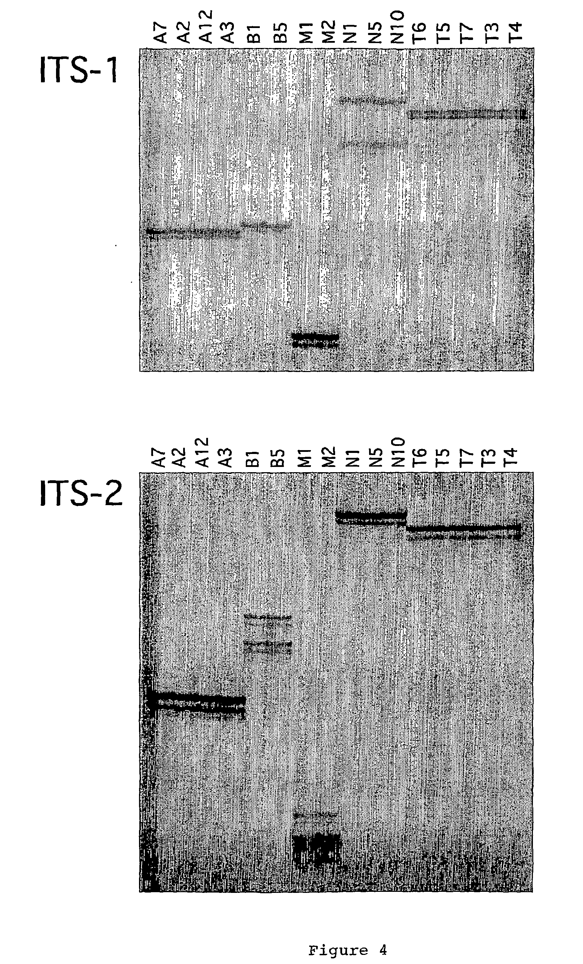

- **Figure 4**: DPGE analysis of ITS-1 or ITS-2 PCR products amplified from multiple oocyst isolates representing five species of *Eimeria* from chickens, in the following order: *E. acervulina* (isolates A7, A2, A12 and A3), *E. brunetti* (isolates B1 and B5), *E. maxima* (isolates M1 and M2), *E. necatrix* (isolates N1, N5 and N10) and *E. tenella* (isolates T6, T5, T7, T3 and T4) (cf. Table 1);

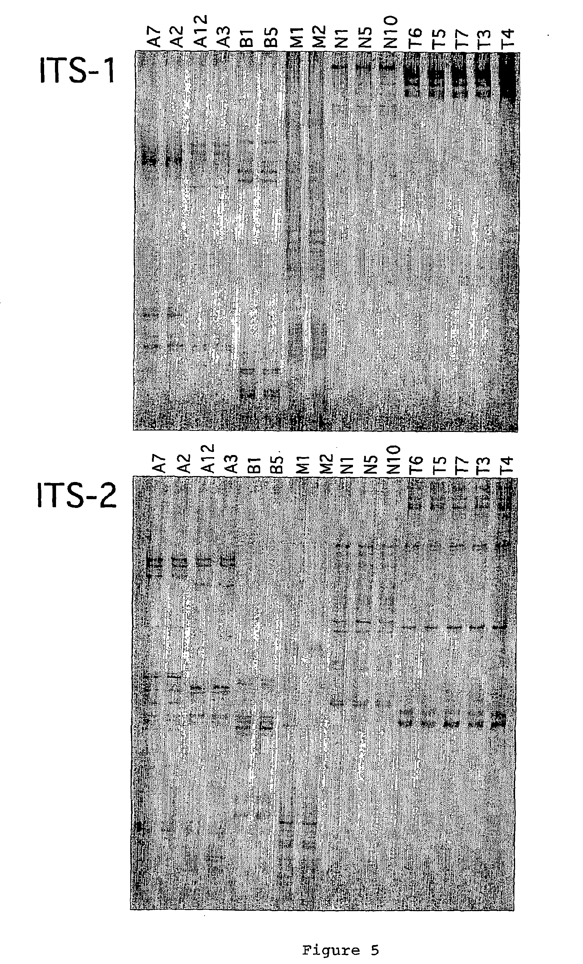

- **Figure 5**: SSCP analysis of ITS-1 or ITS-2 PCR products amplified from multiple oocyst isolates representing five species *of Eimeria* from chickens, in the following order: *E. acervulina* (isolates A7, A2, A12 and A3), *E. brunetti* (isolates B1 and B5), *E. maxima* (isolates M1 and M2), *E. necatrix* (isolates N1, N5 and N10) and *E. tenella* (isolates T6, T5, T7, T3 and T4) (see Table 1);

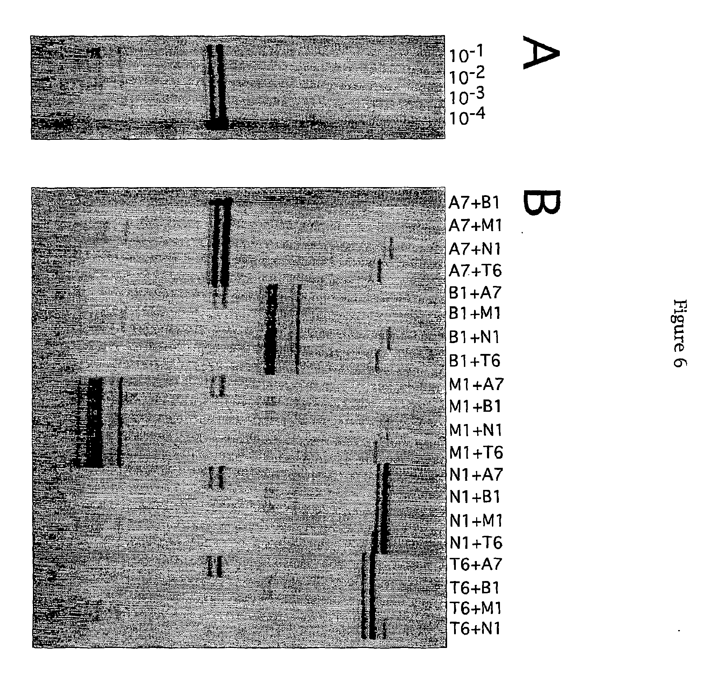

- **Figure 6**: Ability of the DPGE approach to specifically detect multiple species of *Eimeria* within samples. Panel A shows the detection of *Eimeria maxima* in the presence of excess *E. acervulina* (DNA of isolates A7 to M1 mixed in ratios of 1: 10⁻¹, 1:10⁻², 1: 10⁻³ and 1:10⁻⁴). Panel B shows the detection of a particular species (second isolate) in the presence (+) of 100x excess DNA of a heterologous species (first isolate), for all possible species combinations presented. *E. acervulina, E. brunetti, E. maxima, E. necatrix* and *E. tenella* represented by isolates A7, B1, M1, N1 and T6, respectively (see Table 1);

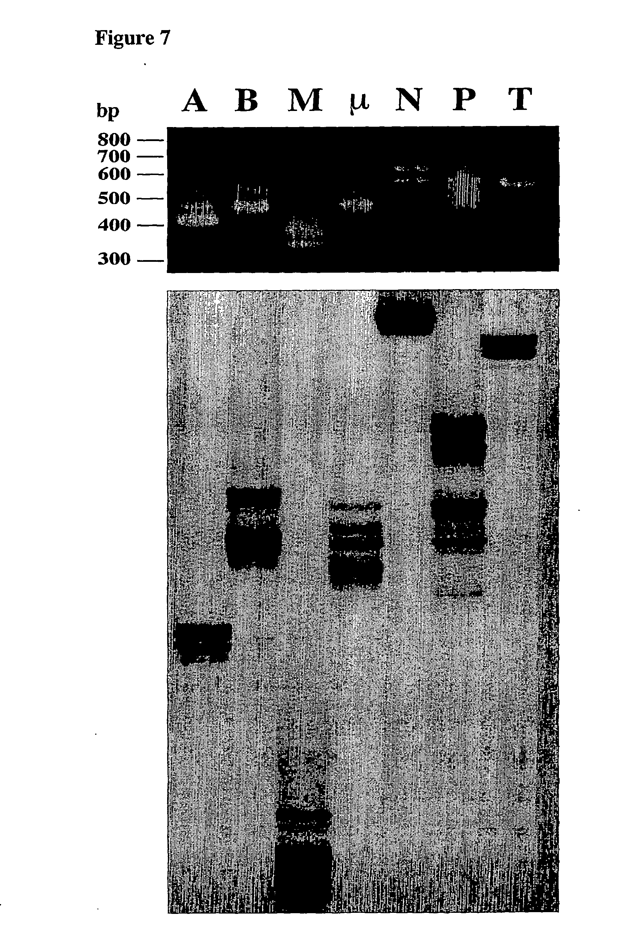

- **Figure 7**: Agarose gel (top) and DPGE gel (bottom) showing ITS-2 PCR products amplified from oocyst isolates representing seven different species of *Eimeria* from chickens. Lanes: *E. acervulina, E. brunetti, E. maxima, E. mitis, E. necatrix, E. praecox* and *E. tenella* (A, B, M, µ, N, P and T, respectively) (cf. Figures 3 and 4). Isolates represented are A7, B1, M1, µ2, N1, P4 and T6 (cf. Table 1);

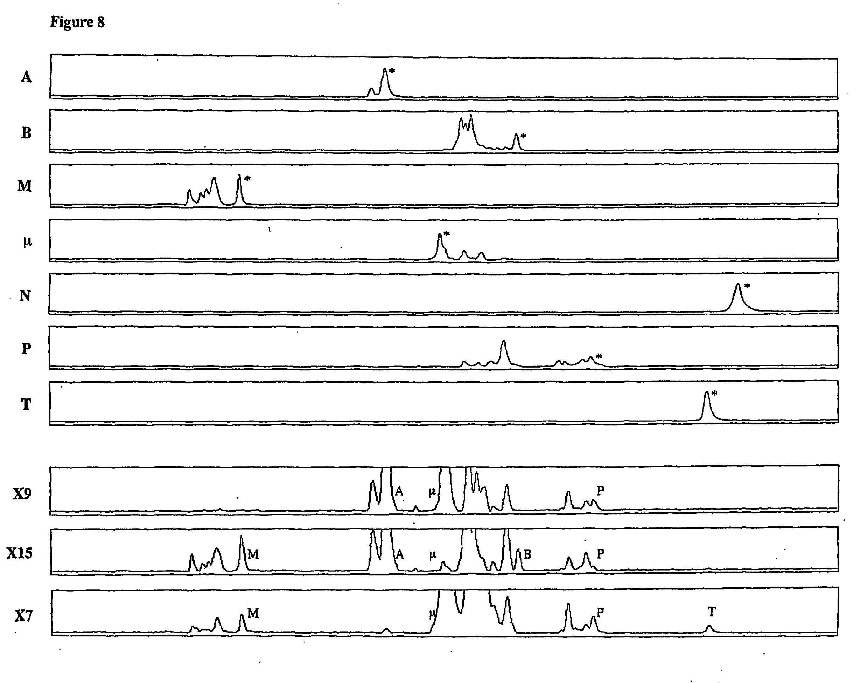

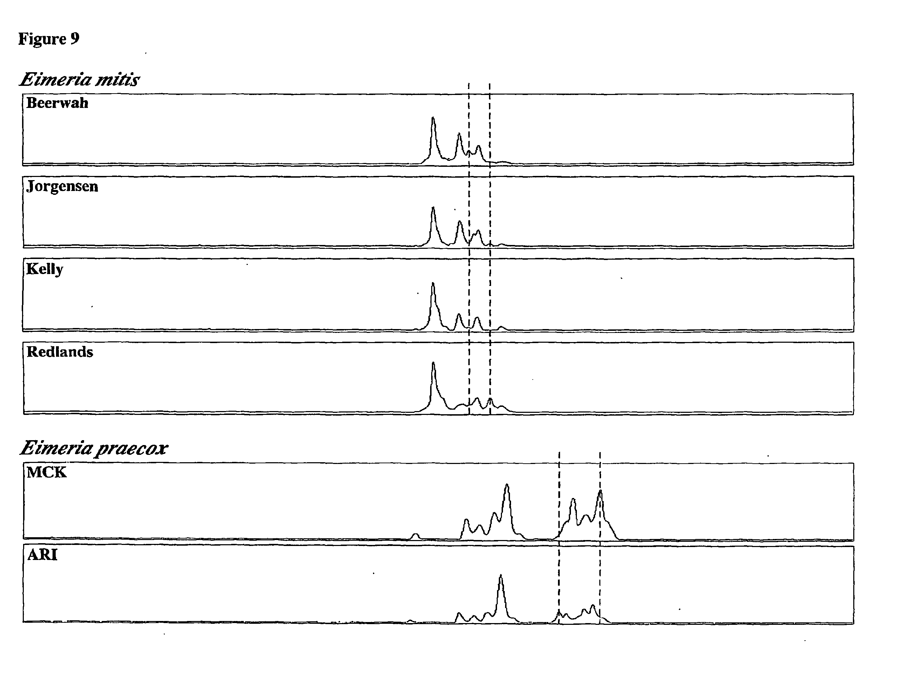

- Figure 8: Chromatograms representing fluorescently-labelled ITS-2 PCR products amplified from seven species of *Eimeria* from chickens (lanes A, B, M, µ, N, P and T representing *E. acervulina, E. brunetti, E. maxima, E. mitis, E. necatrix, E. praecox* and *E. tenella,* respectively) and three Australian isolates from farm chickens containing mixed species of *Eimeria* (lanes X9, X15 and X7, cf. Table 2). The products were separated on a DPGE gel in a 377 automated DNA sequencer (Applied Biosystems, USA) and the chromatograms generated by GeneScan® 3.1 software. For each *E*. *acervulina, E. brunetti, E. maxima, E. necatrix* and *E. tenella,* no appreciable differences in the number and location of peaks in chromatogram profiles were detected between/among samples (not shown), although some variation in relative peak intensity was detected between some samples representing a species. For both *E. mitis* and *E. praecox,* some differences in the positions of minor peaks, indicative of population variation, were detected (cf. Fig. 9). While there was some overlap in profiles among *E. brunetti, E. mitis* and *E. praecox,* species-specific chromatographic peaks could be defined for each of the seven species of *Eimeria* (asterisks), facilitating the identification of the species present in the "farm isolates" (labels A, B, M, µ, P and T signifying peaks representing *E. acervulina, E. brunetti, E. maxima, E. mitis, E. praecox* and *E. tenella,* respectively, cf. Table 2). Monospecific isolates represented are A7, B1, M1, µ2, N1, P4 and T6 (cf. Table 1); and
- **Figure 9**: Chromatograms representing fluorescently-labelled ITS-2 PCR products amplified from different lineages of *E. mitis* and *E. praecox.* The products were separated on a DPGE gel in a 377 automated DNA Sequencer (Applied Biosystems, USA) and the chromatograms generated by GeneScan® 3.1 software. For both *E. mitis* and *E. praecox,* variation in the positions of minor
peaks, indicative of population variation, were detected (vertical lines). The *E. mitis* lineages represented are: Beerwah, Jorgensen, Kelly and Redlands (isolates µ3, µ4, µ2 and µ5, cf. Table 1), and the *E. praecox* lineages are: MCK, and ARI (isolates P2 and P4, cf. Table 1).

### PREFERRED EMBODIMENT

The following is a description of the preferred forms of the present invention given in general terms in relation to the application of the novel oligonucleotides herein described to a method of identifying species of *Eimeria.* The invention is further elucidated from the disclosure given under the sub-heading "Experimental Basis of the Invention" below, which provides examples of preferred forms of the invention.

While the invention described herein is primarily directed to the use of the novel oligonucleotides as primers in genus-specific PCRs, it will be readily appreciated that any of the oligonucleotides described herein may be used in alternative ways to the end of identifying species of *Eimeria.*

Throughout this specification, unless the context requires otherwise, the word "comprise", and variations such as "comprises" and "comprising", will be understood to imply the inclusion of a stated integer or step or group of integers or steps but not the exclusion of any other integer or step or group of integers or steps.

As used herein "genus" is used to refer to a principal rank in the taxonomic hierarchy, falling below the family level and above the species level; "species" is used to refer to a fundamental rank in the taxonomic hierarchy falling below the genus level and indicating the limit of organisms able to interbreed; and "strain" is used to refer to a taxonomic level below the species level, which may indicate population variation within a species. Accordingly, PCRs referred to as "species-specific" are those which are adapted to amplify designated regions of DNA from a single species only. Those PCRs referred to as "genus-specific" are adapted to amplify designated regions of DNA from a number of species within a particular genus.

In a preferred form of the present invention, faecal samples obtained from chickens are processed such that the genomic DNA of any parasitic element present in the sample is isolated. It will be appreciated that any method suitable for the isolation of genomic DNA of *Eimeria* may be used, however, a preferred method is described under the heading "Experimental Basis of the Invention; 1 Parasites and Isolation of Genomic DNA".

Primarily, DNA isolated as above mentioned is subsequently used in the invention as a template in a PCR using either of the novel PCR primer pairs of the invention; WW1 (SEQ ID NO: 25) and WW3r (SEQ ID NO: 26), or, WW2 (SEQ ID NO: 27) and WW4r (SEQ ID NO: 28). The sequences of each of these primers is provided under the sub-heading "Experimental Basis of the Invention; 2 Enzymatic amplification of rDNA" and the position of each of the primers in relation to ITS 1 and ITS2 of *Eimeria* rDNA is depicted in Figures 1 and 2, respectively. The primer pairs of the present invention enable genus-specific PCR amplification of *Eimeria* ITS DNA and thus each pair is applicable to any sample of suspected coccidia and the identification of any number of different species within the genus *Eimeria.*

According to the invention, a PCR primer (or, an oligonucleotide primer) is an oligonucleotide capable of specific hybridisation under particular PCR conditions to a region of the template DNA, which has a sequence which is substantially complementary to the primer sequence, and is adapted to prime the extension of DNA during PCR. It will be realised that a complementary sequence is capable of forming Watson-Crick bonds with its complement, in which adenine pairs with thymine or guanine pairs with cytosine. Each primer is typically used as a member of a primer pair, including a 5' upstream primer that hybridises with the 5' end of the template DNA to be amplified and a 3' downstream primer that hybridises with the complement of the 3' end of the template DNA to be amplified.

Those of ordinary skill in the art to which the invention relates will understand that the term "substantially complementary", as used herein, means that the primer may not have 100% complementarity to its target template sequence but is still capable of annealing thereto in a specific manner under appropriate PCR annealing conditions.

The primers of the present invention may be prepared by any number of conventional DNA synthesis methods. In the present case, the primers were manufactured and purchased commercially from GeneWorks Pty Ltd, PO Box 11, Rundle Mall, Adelaide, SA 5000, Australia.

In accordance with the preferred embodiment, optimal results have been obtained using primers which are identical in length and sequence to the primers WW1 and WW3r and/or, WW2 and WW4r as abovementioned. However, a person of ordinary skill in the art will recognise that alterations may be made to the primers while still maintaining the genus-specificity of the PCR amplification and the efficacy of the present inventive diagnostic method.

Firstly, the length of the primers used may be varied. For example, the present invention contemplates that shorter primers containing at least 15 consecutive bases of the nucleotide sequences of these primers may be suitable. Similarly, the primers may be lengthened. The exact upper limit of the length of the primers is not critical. However, typically primers will be less than or equal to approximately 30 bases and preferably less than or equal to 26 bases. By way of example, it is considered that primer WW1 may be extended by up to 10 nucleotides from its 3' end (TCT AAA GGA T), (SEQ ID NO: 3), WW2 may be extended by up to 4 nucleotides at its 5' end (CAGC), (SEQ ID NO: 4), WW3r may be extended by up to 5 nucleotides from its 3' end (GTT TT), (SEQ ID NO: 5) or up to 10 nucleotides from its 5' end (ATG CGT GAG C), (SEQ ID NO: 6) and WW4r may be extended by up to 10 nucleotides at either end (3' end, ACT GAT TTC A, (SEQ ID NO: 7) and 5' end, TGA TAT GCT T, (SEQ ID NO: 8)). In addition, non-complementary nucleotide fragments may also be attached to the 5' end of the primers, effectively increasing their length.

Secondly, the present invention contemplates minor changes (or conservative alterations) to the sequence of the primers which do not substantially alter their ability to anneal to their specific target DNA and subsequently prime extension during PCR. For example, any particular nucleotide, or plurality of nucleotides, of a primer may be substituted for alternative nucleotides, which may not allow for Watson-Crick base-pairing at the particular site of alteration on annealing of the primer to the template DNA during PCR, but nonetheless does not substantially affect the ability of the primer to prime extension during PCR. Such alternative primers may be referred to as "annealing equivalents" of the primers WW1, WW3r, WW2 or WW4r and variants thereof, as described herein. Such annealing equivalents will be at least 15 nucleotides in length and adapted to anneal to a target sequence under appropriate PCR annealing conditions. Generally, appropriate PCR annealing conditions for such annealing equivalents include the use of a PCR reaction mix or buffer having 3-7mM MgCl₂. It is considered that annealing temperatures of between 45° C and 52°C may be appropriate for most annealing equivalents. By way of further exemplification, if 5 nucleotides within a particular primer sequence were altered in a manner described in this paragraph, and those alterations were spread centrally across the primer sequence, the preferred annealing temperature of that primer is likely to drop by approximately 5°C. A target sequence, as referred to in this paragraph, means a sequence being complementary to at least 15 consecutive bases of the sequence of either one of the primers WW1, WW3r, WW2 or WW4r.

It will be appreciated that the usefulness of any alternative PCR primer sets designed around WW1 and WW3r, and/or, WW2 and WW4r, of the present invention, may be evaluated, at least notionally, using appropriate software and the ITS-1 and ITS-2 and flanking region DNA sequence information. Such software packages include, for example, PC Oligo5 (National Bioscience Inc) or Amplify (University of Wisconsin).

Examples of variations on the primers WW1 , WW3r, WW2 and WW4r which may be appropriate in the present invention include:

**Table 1A**

| **Variations of WW1 (5'-3')** | **Variations of WW3r (5'-3')** |
|---|---|
| AGTTGCGTAAATAGAGCCC (SEQ ID NO: 9) | AAGACATCCATTGCTGAAA (SEQ ID NO: 10) |
| AAGTTGCGTAAAAG AGCC (SEQ ID NO: 11) | CAAGACATCCATTGC TGAA (SEQ ID NO: 12) |
| AAGTTGCGTAAATAGAGC (SEQ ID NO: 13) | CAAGACATCCATTGCTGA (SEQ ID NO: 14) |
| TTGCGTAAATAGAGCCC (SEQ ID NO: 15) | GACATCCATTGCTGAAA (SEQ ID NO: 16) |
| | |

| **Variations of WW2 (5'-3')** | **Variations of WW4r (5'-3')** |
|---|---|
| CGTCTGTTTCAGTGTCT (SEQ ID NO: 17) | AATTCAGCGGGTAACCTCG (SEQ ID NO: 18) |
| ACGTCTGTTTCAGTGTC (SEQ ID NO: 19) | AAATTCAGCGGGTAACCTC (SEQ ID NO: 20) |
| ACGTCTGTTTCAGTG CT (SEQ ID NO: 21) | AAATTCAGCGGGTAACCT (SEQ ID NO: 22) |
| GTCTGTTTCAGTGTCT (SEQ ID NO: 23) | TTCAGCGGGTAACCTCG (SEQ ID NO: 24) |

While the novel oligonucleotides or primers and variants thereof disclosed herein have been designed to enable genus-specific PCR amplification of regions of ITS-1 and ITS-2, it will be appreciated that they may also be applied, individually or in combination, to various other applications. For example they may be used as molecular probes, or primers for alternative diagnostic techniques (such as LCR, ligase chain reaction, or quantitative PCR). Those skilled in the art will readily appreciate the means by which such applications may be effected using standard methodology in the art.

Generally, only one PCR, using a single primer set, will be needed in order to identify the species of *Eimeria* present within a sample. However, it will be appreciated that there may be times where a parallel PCR, using the second primer set, may be utilised in order to further clarify the identity of a species present within a sample. Similarly, upon optimisation of the PCR conditions both novel primer sets may be used in a single PCR.

In order that the PCR products may subsequently be detected, the primers are preferably end-labelled with [γ-³³P]ATP. Alternatively, other means of labelling the PCR products may be utilised; for example, incorporation of [α-³²P]dNTPs during PCR amplification. More preferably, non-radioactive labelling systems using digoxygenin, biotin and the like, may be employed. Such non-radioactive labelling systems are well known in the art and accordingly may be readily applied by a person of ordinary skill. However, one example is provided herein under the heading "Experimental Basis for the Invention - 7 Example of use of Fluorescence-based Electrophoretic Detection". It will be appreciated that the non-radioactive label may be used to end-label a particular primer or may be incorporated into the primer and/or PCR product.

Each PCR is run with at least one monospecific control sample or standard of known species identity. It will be appreciated that control samples containing more than one known species may be entertained. Negative controls in which no *Eimeria* template DNA is present are also run against the samples. It will be appreciated that other standard controls routinely used in the art may also be run against the samples.

The *Eimeria* genomic DNA of the control or standards of known *Eimeria* species may be purified in a like manner to the genomic DNA of unknown samples. Laboratory lines of known *Eimeria* identity obtained from Medichick Laboratories (Australia) or Animal Research Institute of the Queensland Department of Primary Industries (Australia), for example, may be utilised for this purpose.

Amplification is conducted according to conventional procedures in the art to which this invention relates; such as described in US Patent No 4,683,202. Preferably standard PCRs according to the invention include 0. 1µM-1µM of each primer, 200µM each dNTP, 3-7mM MgCl₂, and 1U *Taq* DNA polymerase (Promega). Typically, each PCR is overlayed with mineral oil or the like to prevent evaporation of the reaction mix during cycling. PCR cycling is preferably run under the following conditions: denaturation at a temperature of 94 °C for approximately 15 to 30 seconds, annealing at a temperature of from 45°C to 60°C for 15 to 30 seconds and extension at a temperature of 72°C for 15 to 30 seconds. Preferably between 30 and 35 cycles are run. More specifically, the following PCR conditions may be for the preferred pairs of primers, WW1 and WW3r, and WW2 and WW4r, of the invention:

| **Denaturation** | **Annealing** | **Extension** | **Cycles** |
|---|---|---|---|
| 94°C, 30 seconds | 45°C, 30 seconds | 72°C, 30 seconds | 30 |
| 94°C, 30 seconds | 50°C, 30 seconds | 72°C, 30 seconds | 30 |
| 94°C, 30 seconds | 60°C, 30 seconds | 72°C, 30 seconds | 30 |
| 94°C, 15 seconds | 60°C, 15 seconds | 72°C, 30 seconds | 30 |

It will be appreciated by those of ordinary skill in the art that the PCR conditions provided herein are merely exemplary and may be varied so as to optimise conditions where, for example, alternative PCR cyclers or DNA polymerases are used, where the quality of the template DNA differs, or where variations of the primers not specifically exemplified herein are used, without departing from the scope of the present invention. The PCR conditions may be altered or optimised by changing the concentration of the various constituents within the reaction and/or changing the constituents of the reaction, altering the number of amplification cycles, the denaturation, annealing or extension times or temperatures, or the quantity of template DNA, for example. Those of skill in the art will appreciate there are a number of other ways in which PCR conditions may be optimised to overcome variability between reactions. PCR conditions may be optimised by routine analysis based on the above mentioned factors which are commonly understood in the art.

It will be understood that where not specifically exemplified herein appropriate PCR annealing temperatures for any primer within the scope of the present invention may be derived from the calculated melting temperature of that primer. Such melting temperatures may be calculated using standard formulas, such as that described in Sambrook, 1989. As will be understood by those of ordinary skill in the art to which this invention relates annealing temperatures may be above or below the melting temperature but generally an annealing temperature of approximately 5°C above the calculated melting temperature of the primer may be suitable.

PCR products, obtained from the amplification of regions of ITS-1 and ITS-2 of both unknown samples and relevant control samples or standards of known identity, may be detected by electrophoretic separation. Electrophoretic techniques which are particularly sensitive to minor differences in PCR product size and/or sequence are preferred. For example, the techniques of SSCP (single-strand conformation polymorphism) and/or DPGE (denaturing polyacrylamide gel electrophoresis) are particularly suitable when conditions are optimised as they have the ability to detect single base changes in sequence or variation in length by a single nucleotide between samples. In addition, these techniques are readily applicable to the screening of large numbers of samples.

SSCP analysis has been described (Orita *et al,* 1989). Generally, any particular PCR product may be separated as single-stranded molecules by electrophoresis in a non-denaturing polyacrylamide gel. The technique is based on the fact that a molecule of single-stranded DNA folds differently from another such molecule if it differs in sequence by a single base or more; differences in tertiary structure result in differences in mobility during electrophoresis.

Those of ordinary skill in the art to which this invention relates will understand that the tertiary structure of single-stranded DNA changes under different physical conditions, for example, temperature and ionic environment. As a result, the sensitivity of SSCP depends on these and many other such conditions, such as the length of the PCR product. In the case of the present invention, the following conditions have been found to be preferred, however, it will be appreciated that the conditions may be altered to take account of different laboratory conditions and equipment; 0.4 to 0.6x MDE (mutation detection enhancement; FMC BioProducts) containing 0.5 to 1.5x TBE and electrophoresis performed at 7 to 40W for approximately 17 hours at 15°C. More specifically, the following conditions may be utilised in the invention:

| **Gel** | **Power (W)** | **Time (hours)** | **Temperature (°C)** |
|---|---|---|---|
| 0.5xMDE containing 0.6xTBE | 7 | 17 | 15 |
| 0.4xMDE containing 0.6xTBE | 7 | 17 | 15 |
| 0.6xMDE containing 0.6xTBE | 20 | 17 | 15 |
| 0.6xMDE containing 0.6xTBE | 30 | 17 | 15 |
| 0.6xMDE containing 0.6xTBE | 40 | 17 | 15 |
| 0.6xMDE containing 0.5xTBE | 20 | 17 | 15 |
| 0.6xMDE containing 1.0xTBE | 30 | 17 | 15 |
| 0.6xMDE containing 1.5xTBE | 40 | 17 | 15 |

DPGE has previously been described and is well known in the art to which this invention relates. In DPGE each strand of a DNA molecule is separated from its complementary strand and run on a polyacrylamide gel under denaturing conditions. Under such conditions, the two strands of any particular DNA molecule are prevented from re-hybridising to one another during electrophoresis such that individual strands will migrate separately within the gel. DPGE is a sensitive system which is capable of identifying differences in the length of any two DNA molecules to a single nucleotide.

In the case of DPGE, the following range of conditions are preferable, however, as with the SSCP conditions, they may be altered to take account of many other laboratory variables, without departing from the scope of the present invention; 0.4mm thick gel between 4 and 6% polyacrylamide, containing 42% urea and 1xTBE, subjected to electrophoresis at between 20 to 50W for approximately 4 hours at 40°C. More specifically, the following conditions may be utilised in the present invention:

| **Gel (polyacrylamide)** | **Power (W)** | **Time (Hours)** | **Temperature (°C)** |
|---|---|---|---|
| 0.4mm thick (6%) containing 42% urea and 1xTBE | 40 | 4 | 40 |
| 0.4mm thick (4%) containing 42% urea and 1xTBE | 40 | 4 | 40 |
| 0.4mm thick (5.5%) containing 42% urea and 1xTBE | 40 | 4 | 40 |
| 0.4mm thick (5%) containing 42% urea and 1xTBE | 20 | 4 | 40 |
| 0.4mm thick (5%) containing 42% urea and 1xTBE | 30 | 4 | 40 |
| 0.4mm thick (5%) containing 42% urea and 1xTBE | 50 | 4 | 40 |

It should be noted that DPGE is preferred for species identification while SSCP is preferred for the detection of population variation within a species, although both techniques are applicable to species identification. Thus, it will be appreciated that either one of SSCP or DPGE may be used alone to effect identification or diagnosis of *Eimeria* species. However, the present invention also contemplates both techniques being used in parallel in order to gain a better understanding of the identity of the species of *Eimeria* within a particular sample. Similarly, the present invention considers the use of other complementary techniques such as agarose gel electrophoresis and DNA sequencing.

Following separation of PCR products via electrophoresis, gels may be processed according to standard techniques (for example, in the case of polyacrylamide gels, dried on to filter or blotting paper), and subjected to autoradiography for a time appropriate to be able to demonstrate the position of the PCR product bands on a gel.

The methodology of the present invention is preferably adapted to an automated (fluorescence-based) electrophoretic system; for example, and Applied BioSystems (ABI) automated sequencing apparatus coupled with appropriate computer hard- and software. In this way, specific "fingerprints" for individual species of *Eimeria* may be recorded, stored (for protocol and reporting purposes) and compared against standard samples of known species status. Such electrophoretic methods are standard procedures commonly used and understood by those of general skill in the art. However, specific exemplification of one such system is provided herein under "Experimental Basis of the Invention - 7 Example of Use of Fluorescence-based Electrophoretic detection".

It will be appreciated that where an automated system is used in the present invention that standard conditions generally applicable to manual SSCP and DPGE, as described and exemplified herein, may need to be varied in order to accommodate the particular automated system utilised. Appropriate conditions may be easily determined from manufacturer's instructions and routine optimisation assays according thereto and as are commonly used in the art.

### Experimental Basis of the Invention

### 1 Parasites and isolation of genomic DNA

Australian isolates of *Eimeria* (representing monospecific lines; Table 1) were passaged in specific pathogen free (SPF) chickens maintained in custom-built isolators under stringent conditions to prevent cross-contamination. Isolates were identified to species based on the morphometry of sporulated oocysts, prepatent period and location of gross lesions in the intestine(s). To rule out putative contamination of isolates with one or more heterologous species, the 18S rRNA gene, which also provides species-level identification (Barta *et al,* 1997), was sequenced from PCR products derived from selected oocyst isolates (see 4 below). The 18S sequences determined for individual species had 99-100% identity with those published recently by other workers (Barta et al, 1997).

For each isolate, faeces were collected from groups of chickens and the *Eimeria* oocysts allowed to sporulate under constant aeration at 30°C for a minimum of 48 hours. Oocysts were isolated using saturated NaCl (Shirley *et al,* 1995), washed extensively in 50 ml volumes of H₂O and made up to a final aqueous suspension (10 ml containing 5x10⁶ oocysts). The oocysts were then purified using a sucrose-gradient centrifugation method (Gasser *et al,* 1987), which removed faecal components inhibitory to the PCR, washed (as above) and then resuspended in 1 ml of H₂O. DNA was isolated from oocysts using a Wizard® Genomic DNA Purification Kit (Promega, WI, USA). In brief, each aqueous suspension of oocysts was transferred to a 1.5 ml Eppendorf tube, centrifuged at 13000g for 5 min and resuspended in 300 µl of Nuclei Lysis Solution (Promega, WI, USA). An equal volume of glass beads (2 mm in diameter) was added and the tube vortexed vigorously for 3-5 min until >90% of the oocysts ruptured (assessed by light microscopic examination of a tiny sub-aliquot at 400x magnification). The suspension containing sporocysts was then transferred to a fresh Eppendorf tube, the glass beads discarded, proteinase K (150 µg ml⁻¹) and sodium dodecyl-sulfate (5%w/v) added, and then incubated at 37°C until >90% of the sporocysts had ruptured (-4 h). This lysate was then centrifuged at 13000g for 5 min to pellet the oocyst and sporocyst walls, and the supernatant transferred to a fresh tube. The DNA was purified from the supernatant according to the "yeast DNA protocol" (Promega) and eluted into 50µl of H₂O. Individual samples were checked on ethidium bromide-stained 2% agarose-Tris-Borate-EDTA (TBE = 65 mM Tris-HCl, 27 mM boric acid, 1mM EDTA, pH 9; Bio-Rad, Richmond, CA, USA) gels using specific dilutions of phage λ DNA (Promega, WI, USA) as markers, which were also used to approximate DNA concentrations. The amounts of genomic DNA isolated from individual isolates (- 5 x 10⁶ oocysts) were estimated at 1-2.5 µg.

Table 1. Isolates representing different species of *Eimeria.* All isolates were derived from chicken flocks in Australia and were maintained as monospecific lines. The MCK lines were provided by Medichick Laboratories (Australia); the others were obtained from the Animal Research Institute of the Queensland Department of Primary Industries (Australia).

| Isolate | Species | Description and geographical origin in Australia |
|---|---|---|
| A7 | *E. acervulina* | Attenuated RA line derived from a Queensland flock [11] |
| A2 | *E. acervulina* | 6th passage of A7 |
| A12 | *E. acervulina* | 15 year old lab line (MCK) derived from a Victorian flock |
| A3 | *E. acervulina* | 1st passage of A12 |
| B1 | *E. brunetti* | Attenuated line (AM) derived from a Queensland flock |
| B5 | *E. brunetti* | 5th passage of B1 |
| M1 | *E. maxima* | 15 year old lab line (MCK) derived from a Victorian flock |
| M2 | *E. maxima* | 2nd passage of M1 |
| µ2 | *E. mitis* | Kelly line derived from a Queensland flock |
| µ3 | *E. mitis* | Beerwah line derived from a Queensland flock |
| µ4 | *E. mitis* | Jorgensen line derived from a Queensland flock |
| µ5 | *E. mitis* | Redlands line derived from a Queensland flock |
| N1 | *E. necatrix* | 15 year old lab line (MCK) derived from a Victorian flock |
| N5 | *E. necatrix* | 2nd passage of N1 |
| N10 | *E. necatrix* | 8th passage of attenuated derivative of N1 |
| P2 | *E. praecox* | MCK line derived from a Victorian flock |
| P4 | *E. praecox* | ARI line derived from a Queensland flock |
| T6 | *E. tenella* | Field isolate (DARRYL) from Queensland |
| T5 | *E. tenella* | 4th passage of T6 |
| T7 | *E. tenella* | Attenuated laboratory line (Rt3+15) |
| T3 | *E. tenella* | 4th passage of T7 |
| T4 | *E. tenella* | 15 year old lab line (MCK) derived from a Victorian flock |

### 2 Enzymatic amplification of rDNA

Oligonucleotide primers were designed to regions of the 18S, 5.8S and 28S rRNA gene sequences considered to be specific for the genus *Eimeria.* The ITS-1 (plus flanking sequence) was amplified by PCR using the primers WW1 (forward: 5'-AAG TTG CGT AAA TAG AGC CC-3') and WW3r (reverse: 5'-CAA GAC ATC CAT TGC TGA AA-3'), while ITS-2 was amplified using the primers WW2 (forward: 5'-ACG TCT GTT TCA GTG TCT-3') and WW4r (reverse: 5'-AAA TTC AGC GGG TAA CCT CG-3'). The 18S gene was amplified using primers WW5 (5'- ACC TGG TTG ATC CTG CCA G-3'), (SEQ ID NO: 29), and WW6r (5'-CTT CCG CAG GTT CAC CTA CGG-3'), (SEQ ID NO: 30). Primers used to amplify ITS-1 or ITS-2 were endlabelled with [γ-³³P]ATP (NEN Life Science Products) using T4 polynucleotide kinase according to the manufacturer's protocol (Promega, WI, USA). PCR reactions were performed in 30 µl volumes using -50 ng of template, 50 pmol primer, 200 µM of each dNTP, 7 mM MgCl₂ (for ITS-1) or 3 mM MgCl₂ (for ITS-2) and 1 U Taq DNA polymerase (Promega, WI, USA) under the following thermocycling conditions: 94°C, 30 s (denaturation); 55°C, 30 s (annealing); 72°C, 30 s (extension) for 30 cycles in a DNA Thermal Cycler 480 (Perkin Elmer, USA). Control samples without DNA were included in each PCR run. Also, the specificity of the PCR for both primer sets was tested using DNA (~100 ng) from chicken musculature and faeces (known to be free of *Eimeria),* and no PCR products were detected in any of those control samples after autoradiography of DPGE gels for 24 h.

Individual PCR products were mixed with an equal volume of loading buffer (10 mM NaOH, 95% formamide, 0.05% bromophenol blue and 0.05% xylene cyanol) and their intensity verified on ethidium bromide-stained 2.5% agarose-TBE gels using 100 bp ladder (Promega, WI, USA) as a size marker. The lowest amount of *Eimeria* DNA required for effective amplification (for both primer sets) and visual detection on agarose gels was -5 pg (represents ~5-50 oocysts), which is comparable with previous studies (Stucki *et al,* 1993; Schnitzler *et al,* 1998; Molloy *et al,* 1998).

### 3 High resolution electrophoresis

PCR products were denatured at 95°C for 5 min and snap-cooled on a freeze block (-20°C) for 2 min before loading on to gels. For DPGE, 5 µl of each sample were loaded into the wells of a 0.4 mm thick 5% polyacrylamide gel containing 42% urea and 1 x TBE, and subjected to electrophoresis at 40 W for 4 h at 40°C. For single-strand conformation polymorphism (SSCP), 3 µl of each sample were loaded into the wells of a 0.4 mm thick non-denaturing gel (0.6x MDE™, mutation detection enhancement; FMC BioProducts, Rockland, ME, USA) containing 0.6x TBE, and electrophoresis performed at 7 W for 17 h at 15°C. Optimization was as described previously (Zhu and Gasser, 1998). Both electrophoretic procedures were performed in a conventional sequencing rig (BaseRunner; IBI, New Haven, CT, USA). Gels were dried on to blotting paper and subjected to autoradiography using RP1 film (Agfa).

### 4 DNA sequencing

PCR products were purified over Wizard® PCR Preps columns (Promega, WI, USA) and eluted into 40 µl H₂O. To sequence the 18S rRNA gene, an aliquot (1 µl) was subjected directly to cycle-sequencing with the *fmol®* DNA Cycle Sequencing System (Promega, WI, USA) using the same primers as for PCR and an annealing temperature of 55°C. To sequence the ITS-1, an aliquot of column-purified PCR product (100 ng) was cloned into the pGEM®-T plasmid vector (Promega, WI, USA), and 12 clones (per PCR product) isolated for cycle-sequencing.

### 5 Results

On agarose gels (Fig. 3), the sizes of ITS-1 PCR products varied from -450-770 bp, whereas those for the ITS-2 were -370-620 bp (Table 2). For both ITS-1 and ITS-2, the band size(s) were unique to each species as no intraspecific variation in band profiles was detected between or among multiple isolates. For ITS-1 PCR products, one band was detected for *E. acervulina* and *E. tenella,* whereas 2-3 bands were resolved for the other 3 species (Table 2). For ITS-2 PCR products, one band was detected for *E*. *tenella,* while 2 bands were displayed for all other species (Table 2). The resolution of multiple ITS bands on agarose gels for some species indicated the existence of different sequence types within a PCR product. This was confirmed by sequencing (via cloning) of the ITS-1 PCR products for selected samples representing each species (A7, B1, M1, N1 and T6, Table 1) and comparison with previously published sequences (Schnitzler *et al,* 1998; Schnitzler *et al,* 1999; Barta *et al,* 1998). Sequencing (of 12 clones per species) showed that individual ITS-1 PCR products represented the appropriate species, although novel ITS-1 sequence types (not shown) were detected for *E. brunetti, E. maxima* and *E. necatrix.*

DPGE and SSCP were then evaluated for their ability to display size or sequence variation in denatured PCR products. Both techniques allowed the unequivocal identification of all *Eimeria* species using either ITS-1 and/or ITS-2 PCR products (Figs. 4 and 5). For DPGE (Fig. 4), the banding profiles were relatively simple, each consisting of 2-5 single-strand bands, with no detectable differences in the number or size(s) of bands between multiple isolates of the same species. As expected based on results of the agarose gel electrophoresis (Table 2), no bands representing a particular species were shared by the heterologous species. Although the ITS-1 banding profiles for *E. acervulina* and *E. brunetti* were similar, the two species could be more readily distinguished using ITS-2. Conversely, *E. necatrix* and *E. tenella* were more easily differentiated using the ITS-1 rather than ITS-2 profile. For SSCP (Fig. 5), the banding profiles were relatively complex, each consisting of -6-15 single-strand bands (depending on ITS region and species). The complex profiles are the result of the formation of multiple conformational types of single-stranded molecule(s). No variation in ITS-1 or ITS-2 profiles was detected among multiple isolates representing the same laboratory line for any of the five species (c.f. Table 1). Similarly, no differences were detected among multiple different laboratory lines for both *E. necatrix* and *E. tenella.* In contrast, a significant difference in ITS-1 and ITS-2 profiles was detected between isolates representing the RA and MCK lines of *E*. *acervulina,* which remained undetected by DPGE (Fig. 1; lanes A7 and A2 *versus* lanes A12 and A3). This difference related to polymorphism (or ~1% difference) in both the ITS-1 and ITS-2 sequences between these laboratory lines (unpublished).

The results illustrated in Figure 7 further exemplify the efficacy of using a DPGE approach according to the invention.

**Table 2. Number and approximate sizes of ITS-1 and ITS-2 PCR products for Eimeria species. Sizes determined in 2.5% agarose in 0.5x TBE by comparison with known size standards. PCR product sizes were interpolated using a formula derived by linear regression of molecular weight standards compared with the log of their migration distance.**

| **ITS-1.** | | |
|---|---|---|
| Species | Number of bands | Approximate sizes of bands (bp) |
| *E. acervulina* | 1 | 520 |
| *E. brunetti* | 2 | 530, 580 |
| *E. maxima* | 3 | 450, 560, 610 |
| *E. necatrix* | 3 | 630, 715, 770 |
| *E. tenella* | 1 | 700 |

| **ITS-2.** | | |
|---|---|---|
| Species | Number of bands | Approx. sizes of bands (bp) |
| *E. acervulina* | 2 | 440, 490 |
| *E. brunetti* | 2 | 470, 550 |
| *E. maxima* | 2 | 370, 420 |
| *E necatrix* | 2 | 590, 620 |
| *E. tenella* | 1 | 580 |

### 6 Specificity of Assay - Multiple Species of Eimeria per Sample

As chickens naturally exposed to *Eimeria* may simultaneously harbour more than one species of *Eimeria* (McDougald *et al,* 1997), it is important to determine the capacity of the procedure to specifically detect DNA in samples containing more than one species. This was tested using ITS-2 as an example. To 20 ng of genomic DNA from each species were added: 2ng, 200 pg, 20 pg or 2 pg of DNA from a heterologous species (ratios of 1: 10⁻¹, 1:10⁻² 1: 10⁻³ or 1:10⁻⁴). The DNA template mixes were then subjected to PCR, the PCR products analysed by DPGE and the gels exposed to autoradiographic film (96 h). As an example, the results for *E*. *acervulina* mixed with differing ratios of *E. maxima* are shown in Fig. 6A. For a template ratio of 1:10⁻⁴, a band representing the first species was detected, while using a template ratio of 1:10⁻³ faint bands representing the second species were detectable for most samples (not all results shown). At the dilution ratio of 1:10⁻², the species present at the lower genomic DNA concentration was detected for all combinations (Fig. 6B). These results indicated that the DNA of a particular species was detectable by PCR, even in the presence of excess (100-1000 times the amount) of template from a heterologous species. In addition, DPGE of ITS-2 has been used (in a blind test) to correctly identify all species present in mixed oocyst isolates, indicating that this technique is useful for detecting mixed-species infections in chickens.

### 7 Example of Use of Fluorescence-based Electrophoretic Detection

*Eimeria* oocysts were allowed to sporulate in the faecal samples under constant aeration at 30°C for a minimum of 48 h. They were then isolated using saturated NaCl (Shirley, 1995), washed extensively in 50ml volumes of H₂O and made up to a final aqueous suspension (10ml containing 5x10⁶ oocysts). The oocysts were then purified over a sucrose-gradient (Gasser et al., 1987), which removed faecal components inhibitory to the PCR, washed and then resuspended in 0.5 ml of H₂O. An equal volume of glass beads (2mm in diameter) was added and the tube mixed vigorously for 3-5min until >90% of the oocysts ruptured (assessed by light microscopic examination at 400x magnification). The suspension containing sporocysts was then transferred to a fresh Eppendorf tube, proteinase K (150µg ml⁻¹) and sodium dodecyl-sulfate (5% w/v) added, and then incubated at 37°C overnight. Solid material was pelleted by centrifugation at 13000g for 5min, and DNA was isolated from the supernatant using a Wizard® DNA Clean-Up Kit (Promega, WI, USA).

The ITS-2 region (plus flanking sequence) was PCR-amplified from individual genomic DNA samples using the primers WW2 and WW4r. Primer WW2 was 5' end-labelled with the fluorescent dye, FAM (Fluorescein). PCR reactions were performed in 25µl volumes usually containing 1-20ng of genomic DNA template, 50pmol primer, 200µM of each dNTP, 3mM MgCl₂ and 1 U Taq DNA polymerase (Promega, WI, USA) under the following thermocycling conditions: 94°C, 15 s (denaturation); 60°C, 15 s (annealing); 72°C, 30 s (extension) for 30 cycles in a DNA Thermal Cycler 2400 (Perkin Elmer). Samples without DNA (no-DNA) or with chicken DNA (purified as for *Eimeria* DNA) were also included as control reactions. The lowest amount of genomic template from which the ITS-2 could be amplified effectively and which was detectable on an agarose gel was estimated at 100 to 1000pg (= X oocyst equivalents), even in the presence of (1000x excess DNA from a heterologous species or from host (i.e. muscle or faecal DNA).

Amplicons were examined and their intensity verified on ethidium bromide-stained 2.5% agarose gels, using 65 mM Tris-HCl, 27 mM boric acid, 1 mM EDTA, pH 9 (TBE) as the buffer and a 100 bp ladder (Promega, WI, USA) as a size marker. Then, each sample was diluted 1/4 with H₂O, and 1.5 µl thereof mixed with 2 µl 4.1 mM EDTA, 88.25% formamide, 15mg/ml dextran blue (Promega) and 0.6 µl of the GeneScan® -2500 [TAMRA] size standard (Applied Biosystems). Samples were denatured at 95°C for 2 min, loaded (2 µl) on to a 5% LongRanger® gel (FMC) in an ABI Prism™ 377 DNA Sequencer (Applied Biosystems) and then subjected to electrophoresis at 200 W (3000V and 60 mA) for 3.5 h. The resultant gel image representing each samples was captured and chromatograms analysed and compared using GeneScan® 3.1 software (Applied Biosystems). Each peak represented the position of each sense strand for each denatured amplicon. The intensity (vertical axis) scale of the chromatograms was adjusted, such that background fluorescence was displayed, and hence no peaks remained undetected.

### 8 Results of Example Study of Use of Fluorescence-based Electrophoretic Detection

Eighteen DNA reference samples representing monospecific isolates of *Eimeria* oocysts were prepared (Table 1). Agarose gel electrophoretic analysis of ITS-2 PCR products amplified (individually) from all monospecific samples revealed that one band was detected for *E*. *tenella,* while two bands were displayed for all other species (not shown). The resolution of multiple bands on agarose gels for some species indicated the existence of different sequence types within an amplicon, which is consistent with previous findings (Woods *et al.,* 2000a,b). This has been confirmed by sequencing of cloned ITS-2 amplicons (3 clones sequenced) for selected samples (A7, B1, M1, N1 and T6) representing each species (see Table 1). Also, the ITS-2 sequences determined for isolates M1 *(E. maxima)* and T6 (E. *tenella)* (Table 1) showed concordance with sequences deposited in GenBank™ (accession numbers AF027722, AF027723, AF027724, AF027725, AF027726 and AF026388). Some novel ITS-2 sequence types have been detected for *E. mitis* and *praecox,* which was not unexpected, given that no study has yet rigorously examined intra-specific or intra-isolate sequence variability in the ITS-2 region.

Amplicons produced from all of the monospecific *Eimeria* DNA samples (Table 1) were then analysed in the automated sequencer. Representative chromatograms are shown in Figure 8. For each *E. acervulina, E. brunetti, E. maxima, E. necatrix* and *E. tenella,* there were no appreciable differences in the number and location of peaks in chromatogram profiles between samples (selected samples shown), although some differences in relative peak intensity were detected between some samples representing a species (Figure 8). For both *E. mitis* and *E. praecox,* some differences in the position(s) of minor peaks, indicative of population variation, were detected (Figure 9). Irrespective of such intraspecific variability in chromatogram profiles, the position of the highest/main peak (arrowed) remained consistent for all samples for each species. While there was some overlap in profiles among *E. brunetti, E. mitis* and *E. praecox,* species-specific chromatographic peaks could be defined for each of the seven species of *Eimeria* (asterisks in Figure 8).

As naturally infected chickens may simultaneously harbour more than one species of *Eimeria,* the ability of the automated fluorescence-based electrophoretic approach to specifically detect DNA in samples containing more than one species was established. Thirteen unknown "field" samples, each derived from groups of multiple chickens, were tested (Table 3). By comparison with reference samples (A6, B1, M1, µ2, N1, P4 and T6), the species compositions within individual samples could be readily determined (Table 3, and Figure 8), even for samples containing *E. brunetti, E. mitis* and *E. praecox,* whose profiles overlapped significantly. While most faecal samples contained multiple species, including *E. praecox and E. mitis* (n =1), *E. acervulina* and *E. mitis* (n=1), *E. acervulina* and *E. praecox* (n=2), *E. acervulina, E. mitis* and *E. praecox* (n=1), *E. acervulina, E. maxima, E. mitis, E. praecox* and *E. tenella* (n=1), *E. acervulina, E. brunetti, E. maxima, E. mitis* and *E. praecox* (n=1), *E. acervulina, E. maxima* and *E.mitis* (n=1), several of samples contained *E. praecox* only (n=4) or *E. mitis* only (n=I). Subsequently, in a blind test, the reproducibility of the method to correctly identify all species present in mixed oocyst isolates as well as in deliberately mixed genomic DNA samples was evaluated. No significant variation in chromatogram profiles was detected among different runs on different days, and the species compositions of mixed samples was as expected. These findings proved that the technique is useful for detecting single- and mixed-species infections in chickens.

**Table 3. Samples of Eimeria obtained from Australian poultry farms. Species composition was determined by DPGE analysis of fluorescently-labelled ITS-2 products (cf. Figure 8).**

| Sample | Species Detected |
|---|---|
| X1 | *E. acervulina, E. praecox* |
| X2 | *E. mitis, E. praecox* |
| X4 | *E. praecox* |
| X5 | *E. mitis* |
| X7 | *E. acervulina, E. maxima, E. mitis, E. praecox, E. tenella* |
| X8 | *E. acervulina, E. maxima, E. mitis* |
| X9 | *E. acervulina, E. mitis, E. praecox* |
| X10 | *E. praecox* |
| X11 | *E. acervulina, E. mitis* |
| X13 | *E. praecox* |
| X14 | *E. acervulina, E. praecox* |
| X15 | *E. acervulina, E. brunetti, E. maxima, E. mitis, E. praecox* |
| X 16 | *E. praecox* |

### ADVANTAGES AND INDUSTRIAL APPLICABILITY

It will be apparent from the description herein that the assay of the present invention has a number of advantages over previous known techniques used to identify species of *Eimeria.* For example, the present assay does not require the use of multiple primer sets, specific to each of the species of *Eimeria* which one wishes to detect, but rather a single genus-specific primer set may be utilised. As a result, multiple species may be co-amplified from a single test sample and run in a single lane on an appropriate electrophoretic gel.

The electrophoretic gel systems (SSCP and/or DFGE) used in the invention to visualise the amplification products, and thus identify the species present within a sample, are extremely sensitive and have the ability to differentiate species which may differ in length by a single nucleotide, or by minor sequence variations.

The fluorescence-based electrophoretic detection technique described herein also has great advantages compared with previous techniques utilised to identify species of *Eimeria,* particularly in combination with the novel primers of the present invention. The use of such a detection system obviates the need to use radioactive nucleotides, and hence is safer for the operator to carry out. Also, reading of electrophoretic gels is performed automatically using a computer imaging system, which eliminates the need for handling and exposure of thereof, and significantly reduces time. Moreover, the chromatograms representing different samples (run on different gels and days) can be recorded and stored electronically as well as retrieved for comparative analysis at any time. Hence, this automated electrophoretic approach is time and cost effective, is well suited for the typing of large numbers of oocyst samples and may provide a useful epidemiological tool for prevalence studies as well as the monitoring of coccidiosis outbreaks.

Further, the invention described herein offers significant advantages over RAPD-PCR (Johnston *et al,* 1995; Greif *et al,* 1996; Shirley *et al,* 1994 (Parasitol Res); Shirley *et al,* 1994 (Res Vet Sci)) in that it employs well-defined primers to a specific region of rDNA for PCR at relatively high stringency, thereby keeping to a minimum problems with the amplification of contaminating host DNA, and with reproducibility as a consequence of non-specificity of primers and the low annealing temperature in PCR (Ellsworth *et al,* 1993; MacPherson *et al,* 1993). Taken together, the above novel features allow for the rapid, high resolution, qualitative screening of large numbers of samples for any species of the genus *Eimeria.* Further, the invention obviates the need to conduct DNA sequence analysis (in the first instance), which reduces time, labour and expense.

The present invention may be used, experimentally or on a commercial scale, as a means of routine diagnosis and monitoring of coccidia, particularly avian coccidia. Alternatively, the invention may be applicable to the quality control of species status of monospecific laboratory lines of *Eimeria.* It may further be useful as a complementary tool in the development of future commercial vaccines and diagnostic tests.

There is intraspecific and interspecific DNA sequence variation within species of *Eimeria.* As a result, prior techniques used to detect species of *Eimeria* have steered clear of utilising anything but species-specific PCRs so as to obviate the need for necessary extensive sequencing and characterisation of resultant PCR products in order to identify isolates, at least at the species level. In contrast one aspect of the present invention utilises genus-specific PCRs which specifically target variation in sequences both within and between species of *Eimeria.* This technique has surprisingly proven to be advantageous.

The invention has been described herein, with reference to certain preferred embodiments, in order to enable the reader to practice the invention without undue experimentation. However, a person having ordinary skill in the art will readily recognise that many of the components and parameters may be varied or modified to a certain extent without departing from the scope of the invention. Furthermore, titles, headings, or the like are provided to enhance the reader's comprehension of this document, and should not be read as limiting the scope of the present invention.

The reference to any prior art in this specification is not, and should not be taken as, an acknowledgment or any form of suggestion that the prior art forms part of the common general knowledge in the field of endeavour.

### BIBLIOGRAPHY:

Andrews, R. H. and Chilton, N. B., *Int. J. Parasitol.* 1999, *29*, 213-253.
Barta, J: R, Martin, D. S., Liberator, P. A., Dashkevicz, M., Anderson, J. W., Feighner, S. D., Elbrecht, A., Perkins-Barrow, A., Jenkins, M. C., Danforth, H.D., Ruff, M. D. and Profous-Juchelka, H., *J. Parasitol.* 1997, *83*, 262-271.
Barta, J. R, Coles, B. A., Schito, M. L., Fernando, M. A., Martin, A. and Danforth, H. D., *Int. J. Parasitol.* 1998, *28*, 485-492.
Eckert, J., Taylor, M., Catchpole, J., Licois, D., Coudert, P. and Bucklar, H., in: Eckert, J., Braun, R, Shirley, M. W. and Coudert, P. (Eds.), *Guidelines on techniques in coccidiosis research,* European Commission, Luxembourg 1995, pp. 103-119.
Ellsworth, D. L., Rittenhouse, K. D. and Honeycutt, R L., *Biotechniques* 1993,*14*, 214-217. Gasser, R. B., Eckert, J. and Rohrer, L., *Parasitol. Res.* 1987, *74,* 103-111.
Gasser, R. B., *Int. J. Parasitol.* 1997, *27*, 1449-1463.
Greif, G., Stephan, B. and Haberkorn, A., *Parasitol. Res.* 1996, *82*, 706-714.
Johnston, D. A. and Fernando, M. A., *Parasitol. Res.* 1995, *81*, 91-97.
Johnston, D. A. and Fernando, M. A., *Parasitol. Res.* 1997, *83*, 464-470.
MacPherson, J. M., Eckstein, P. E., Scoles, G. J. and Gajadhar, A. A., *Mol. Cell. Probes* 1993, *7*, 293-299
McDougald, L. R and Reid, W. M., in: Calnek, B. W., Barnes, H. J., Beard, C. W., McDougald, L. R. and M., S. Y. (Eds.), *Diseases of Poultry,* 10th Edition, Iowa State University Press, Ames, Iowa 1997, pp. 865-883.
Molloy, J. B., Eaves, F. W., Jeston, P. J., Minchin, C. M., Stewart, N. P., Lew, A. E. and Jorgensen, W. K*., Avian Dis.* 1998, *42*, 119-123.
Orita M, Suzuki Y, Sekiya T, Hayashi K, *Genomics,*1989, *5*, 874-879.
Sambrook, J, Fritsch, EF, Maniatis, T, *Molecular cloning: a laboratory manual,* 2nd ed, Cold Spring Harbour Press, 1989
Schnitzler, B. E., Thebo, P. L., Mattsson, J. G., Tomley, F. M. and Shirley, M. W., *Avian Pathol.* 1998, *27*,490-497.
Schnitzler, B. E., Thebo, P. L., Tomley, F. M., Uggla, A. and Shirley, M. W., *Avian Pathol.* 1999, *28*, 89-93.
Shirley, M. W. and Bumstead, N., *Parasitol. Res.* 1994, *80*, 346-351.
Shirley, M. W., *Res. Vet. Sci.* 1994, *57,* 10-14.
Shirley, M. W., in: Eckert, J., Braun, R., Shirley, M. W. and Coudert, P. (Eds.), *Guidelines on techniques in coccidiosis research,* European Commission, Luxembourg 1995, pp. 1-24.
Stucki, U., Braun, R. and Roditi, I., *Exp. Parasitol.* 1993, *76*, 68-75.
Zhu, X. Q. and Gasser, R. B., *Electrophoresis* 1998, *19*, 1366-1373.
Woods, W. G., G. Richards, K. G. Whithear, G. R. Anderson, W. K. Jorgensen, and R. B. Gasser. 2000. High-resolution electrophoretic procedures for the identification of five *Eimeria* species from chickens and detection of population variation. Electrophoresis:in press.
Woods, W. G., K. G. Whithear, D. G. Richards, G. R. Anderson, W. K. Jorgensen, and R. B. Gasser. 2000. Single-strand restriction fragment length polymorphism analysis of the second internal transcribed spacer (ribosomal DNA) for six species of *Eimeria* from chickens in Australia. Int. J. Parasitol. *30*:1019-1023.

## Claims

1. An oligonucleotide comprising at least 15 consecutive bases of the DNA sequence designated SEQ ID NO: 25, or an annealing equivalent thereof, wherein said oligonucleotide is capable of specific hybridisation under PCR conditions to a target sequence being complementary to said at least 15 consecutive bases, and said oligonucleotide is adapted to prime the extension of DNA during PCR.

2. An oligonucleotide as claimed in claim 1 comprising the sequence designated SEQ ID NO: 25.

3. An oligonucleotide sequence as claimed in claim 1 wherein the oligonucleotide is chosen from the group consisting of: SEQ ID NO: 9; SEQ ID NO: 11; SEQ ID NO: 13; and SEQ ID NO: 15.

4. An oligonucleotide comprising at least 15 consecutive bases of the DNA sequence designated SEQ ID NO: 26, or an annealing equivalent thereof, wherein said oligonucleotide is capable of specific hybridisation under PCR conditions to a target sequence being complementary to said at least 15 consecutive bases, and said oligonucleotide is adapted to prime the extension of DNA during PCR.

5. An oligonucleotide as claimed in claim 4 comprising the sequence designated SEQ ID NO: 26.

6. An oligonucleotide as claimed in claim 4 wherein the oligonucleotide is chosen from the group consisting of: SEQ ID NO: 10; SEQ ID NO: 12; SEQ ID NO: 14; and SEQ ID NO: 16.

7. An oligonucleotide comprising at least 15 bases of the DNA sequence designated SEQ ID NO: 27, or an annealing equivalent thereof, wherein said oligonucleotide is capable of specific hybridisation under PCR conditions to a target sequence being complementary to said at least 15 consecutive bases, and said oligonucleotide is adapted to prime the extension of DNA during PCR.

8. An oligonucleotide as claimed in claim 7 comprising the sequence designated SEQ ID NO: 27.

9. An oligonucleotide as claimed in claim 7 wherein the oligonucleotide is chosen from the group consisting of: SEQ ID NO: 17; SEQ ID NO: 19; SEQ ID NO: 21; and SEQ ID NO: 23.

10. An oligonucleotide comprising at least 15 consecutive bases of the DNA sequence designated SEQ ID NO: 28, or an annealing equivalent thereof, wherein said oligonucleotide is capable of specific hybridisation under PCR conditions to a target sequence being complementary to said at least 15 consecutive bases, and said oligonucleotide is adapted to prime the extension of DNA during PCR.

11. An oligonucleotide as claimed in claim 10 comprising a sequence designated SEQ ID NO: 28.

12. An oligonucleotide as claimed in claim 10 wherein the oligonucleotide is chosen from the group consisting of: SEQ ID NO: 18; SEQ ID NO: 20; SEQ ID NO: 22; and SEQ ID NO: 24.

13. A pair of PCR primers, one primer comprising at least 15 consecutive bases of the DNA sequence designated SEQ ID NO: 25, or an annealing equivalent thereof, and a second primer comprising at least 15 consecutive bases of the DNA sequence designated SEQ ID NO: 26, or an annealing equivalent thereof.

14. A pair of PCR primers as claimed in claim 13 wherein said pair of PCR primers comprises one primer comprising a DNA sequence designated SEQ ID NO: 25 and a second primer comprising the DNA sequence designated SEQ ID NO: 26.

15. A pair of PCR primers as claimed in claim 13 wherein said pair of PCR primers comprises one of SEQ ID NO: 9; SEQ ID NO: 11; SEQ ID NO: 13; or SEQ ID NO: 15; and one of SEQ ID NO: 10; SEQ ID NO: 12; SEQ ID NO: 14; or SEQ ID NO: 16.

16. A pair of PCR primers, one primer comprising at least 15 consecutive bases of the DNA sequence designated SEQ ID NO: 27, or an annealing equivalent thereof and a second primer comprising at least 15 consecutive bases of the DNA sequence designated SEQ ID NO: 28, or an annealing equivalent thereof.

17. A pair of PCR primers as claimed in claim 16 wherein said pair of PCR primers comprises a primer comprising the DNA sequence designated SEQ ID NO: 27 and a primer comprising the DNA sequence designated SEQ ID NO: 28.

18. A pair of PCR primers as claimed in claim 16 wherein said pair of PCR primers comprises one of SEQ ID NO: 17; SEQ ID NO: 19; SEQ ID NO: 21; or SEQ ID NO: 23; and one of SEQ ID NO: 18; SEQ ID NO: 20; SEQ ID NO: 22; or SEQ ID NO: 24.

19. A method of identifying *Eimeria* in a sample, said method comprising the steps:
- providing a sample comprising genomic template DNA to be tested;
- providing genomic DNA of one or more standards of known identity;
- providing a pair of PCR primers selected from the group consisting of
(i) primers comprising at least 15 consecutive bases of the DNA sequence designated SEQ ID NO: 25 and SEQ ID NO: 26 or annealing equivalents thereof; or
(ii) primers comprising at least 15 consecutive bases of the DNA sequences designated SEQ ID NO: 27 and SEQ ID NO: 28 or annealing equivalents thereof;
- amplifying by means of PCR a region of template DNA using said primer pair to produce one or more PCR products from said sample, and said one or more standard of known identity;
- comparing said one or more PCR products derived from said sample against one or more PCR products of said one or more standard of known identity; and
- identifying the species of *Eimeria* present within the sample.

20. A method as claimed in claim 19 wherein said pair of PCR primers comprises the primers comprising of sequences designated SEQ ID NO: 25 and SEQ ID NO: 26.

21. A method as claimed in claim 19 wherein said pair of PCR primers comprises one of SEQ ID NO: 9; SEQ ID NO: 11; SEQ ID NO: 13; or SEQ ID NO: 15 and one of SEQ ID NO: 10; SEQ ID NO: 12; SEQ ID NO: 14; or SEQ ID NO: 16.

22. A method as claimed in claim 19 wherein said pair of PCR primers comprises the primers comprising of sequences designated SEQ ID NO: 27 and SEQ ID NO: 28.

23. A method as claimed in claim 19 wherein said pair of PCR primers comprises one of SEQ ID NO: 17; SEQ ID NO: 19; SEQ ID NO: 21; or SEQ ID NO: 23 and one of SEQ ID NO: 18; SEQ ID NO: 20; SEQ ID NO: 22; or SEQ ID NO: 24.

24. A method as claimed in any one of claims 19 to 23 wherein two PCRs are run per sample to be tested, and wherein each PCR uses a different primer pair.

25. A method as claimed in any one of claims 19 to 23 wherein one PCR is run per sample to be tested and both primer pairs are provided within said one PCR.

26. A method as claimed in any one of claims 19 to 25 wherein said one or more PCR products derived from said sample are compared against said one or more PCR products of said one or more standard of known identity by means of gel electrophoresis.

27. A method as claimed in claim 26 wherein the gel electrophoresis is DPGE.

28. A method as claimed in claim 26 wherein the gel electrophoresis is SSCP.

29. A method as claimed in claim 26 wherein both DPGE and SSCP are employed.

30. A method as claimed in anyone of claims 26 and claim 29 wherein gel electrophoresis is conducted in an automated system.

31. The use of any one of the oligonucleotides defined in claims 1 to 12, or annealing equivalents thereof, for the identification of species of *Eimeria.*

## Patentansprüche

1. Oligonucleotid, umfassend wenigstens 15 aufeinander folgende Basen der als SEQ ID NO: 25 bezeichneten DNA-Sequenz, oder ein anlagerndes Äquivalent davon, wobei das Oligonucleotid unter den Bedingungen einer PCR zur spezifischen Hybridisierung an eine zu den wenigstens 15 aufeinander folgenden Basen komplementäre Zielsequenz in der Lage ist und wobei das Oligonucleotid so gestaltet ist, dass es die Verlängerung der DNA während der PCR initiiert.

2. Oligonucleotid nach Anspruch 1, umfassend die als SEQ ID NO: 25 bezeichnete Sequenz.

3. Oligonucleotid-Sequenz nach Anspruch 1, wobei das Oligonucleotid aus der Gruppe bestehend aus SEQ ID NO: 9, SEQ ID NO: 11, SEQ ID NO: 13 und SEQ ID NO: 15 ausgewählt ist.

4. Oligonucleotid, umfassend wenigstens 15 aufeinander folgende Basen der als SEQ ID NO: 26 bezeichneten DNA-Sequenz, oder ein anlagerndes Äquivalent davon, wobei das Oligonucleotid unter den Bedingungen einer PCR zur spezifischen Hybridisierung an eine zu den wenigstens 15 aufeinander folgenden Basen komplementäre Zielsequenz in der Lage ist und wobei das Oligonucleotid so gestaltet ist, dass es die Verlängerung der DNA während der PCR initiiert.

5. Oligonucleotid nach Anspruch 4, umfassend die mit SEQ ID NO: 26 bezeichnete Sequenz.

6. Oligonucleotid nach Anspruch 4, wobei das Oligonucleotid aus der Gruppe bestehend aus SEQ ID NO: 10, SEQ ID NO: 12, SEQ ID NO: 14 und SEQ ID NO: 16 ausgewählt ist.

7. Oligonucleotid, umfassend wenigstens 15 Basen der als SEQ ID NO: 27 bezeichneten DNA-Sequenz, oder ein anlagerndes Äquivalent davon, wobei das Oligonucleotid unter den Bedingungen einer PCR zur spezifischen Hybridisierung an eine zu den wenigstens 15 aufeinander folgenden Basen komplementäre Zielsequenz in der Lage ist und wobei das Oligonucleotid so gestaltet ist, dass es die Verlängerung der DNA während der PCR initiiert.

8. Oligonucleotid nach Anspruch 7, umfassend die als SEQ ID NO: 27 bezeichnete Sequenz.

9. Oligonucleotid nach Anspruch 7, wobei das Oligonucleotid aus der Gruppe bestehend aus SEQ ID NO: 17, SEQ ID NO: 19, SEQ ID NO: 21 und SEQ ID NO: 23 ausgewählt ist.

10. Oligonucleotid, umfassend wenigstens 15 aufeinander folgende Basen der als SEQ ID NO: 28 bezeichneten DNA-Sequenz, oder ein anlagerndes Äquivalent davon, wobei das Oligonucleotid unter den Bedingungen einer PCR zur spezifischen Hybridisierung an eine zu den wenigstens 15 aufeinander folgenden Basen komplementäre Zielsequenz in der Lage ist und wobei das Oligonucleotid so gestaltet ist, dass es die Verlängerung der DNA während der PCR initiiert.

11. Oligonucleotid nach Anspruch 10, umfassend eine als SEQ ID NO: 28 bezeichnete Sequenz.

12. Oligonucleotid nach Anspruch 10, wobei das Oligonucleotid aus der Gruppe bestehend aus SEQ ID NO: 18, SEQ ID NO: 20, SEQ ID NO: 22 und SEQ ID NO: 24 ausgewählt ist.

13. PCR-Primer-Paar, wobei es sich bei einem der Primer um einen Primer handelt, der wenigstens 15 aufeinander folgende Basen der als SEQ ID NO: 25 bezeichneten DNA-Sequenz umfasst, oder um ein anlagerndes Äquivalent davon, und wobei es sich bei dem zweiten Primer um einen Primer handelt, der wenigstens 15 aufeinander folgende Basen der als SEQ ID NO: 26 bezeichneten DNA-Sequenz umfasst, oder um ein anlagerndes Äquivalent davon.

14. PCR-Primer-Paar nach Anspruch 13, wobei das PCR-Primer-Paar einen Primer umfasst, der eine als SEQ ID NO: 25 bezeichnete DNA-Sequenz umfasst, sowie einen zweiten Primer, der eine als SEQ ID NO: 26 bezeichnete DNA-Sequenz umfasst.

15. PCR-Primer-Paar nach Anspruch 13, wobei das PCR-Primer-Paar eine der Sequenzen SEQ ID NO: 9, SEQ ID NO: 11, SEQ ID NO: 13 oder SEQ ID NO: 15, und eine der Sequenzen SEQ ID NO: 10, SEQ ID NO: 12, SEQ ID NO: 14 oder SEQ ID NO: 16 umfasst.

16. PCR-Primer-Paar, wobei es sich bei einem der Primer um einen Primer handelt, der wenigstens 15 aufeinander folgende Basen der als SEQ ID NO: 27 bezeichneten DNA-Sequenz umfasst, oder um ein anlagerndes Äquivalent davon, und wobei es sich bei dem zweiten Primer um einen Primer handelt, der wenigstens 15 aufeinander folgende Basen der als SEQ ID NO: 28 bezeichneten DNA-Sequenz umfasst, oder um ein anlagerndes Äquivalent davon.

17. PCT-Primer-Paar nach Anspruch 16, wobei das PCR-Primer-Paar einen Primer umfasst, der die als SEQ ID NO: 27 bezeichnete DNA-Sequenz umfasst, und einen Primer, der die als SEQ ID NO: 28 bezeichnete DNA-Sequenz umfasst.

18. PCR-Primer-Paar nach Anspruch 16, wobei das PCR-Primer-Paar eine der Sequenzen SEQ ID NO: 17, SEQ ID NO: 19, SEQ ID NO: 21 oder SEQ ID NO: 23, und eine der Sequenzen SEQ ID NO: 18, SEQ ID NO: 20, SEQ ID NO: 22 oder SEQ ID NO: 24 umfasst.

19. Verfahren zur Identifizierung von *Eimeria* in einer Probe, wobei das Verfahren folgende Schritte umfasst:
- Bereitstellen einer Probe, welche die zu testende genomische Original-DNA umfasst;
- Bereitstellen genomischer DNA eines oder mehrerer Standards bekannter Identität;
- Bereitstellen eines PCR-Primer-Paares, das ausgewählt ist aus der Gruppe bestehend aus
(i) Primern, die wenigstens 15 aufeinander folgende Basen der als SEQ ID NO: 25 und SEQ ID NO: 26 bezeichneten DNA-Sequenz umfassen, oder anlagernden Äquivalenten davon; oder
(ii) Primern, die wenigstens 15 aufeinander folgende Basen der als SEQ ID NO: 27 und SEQ ID NO: 28 bezeichneten DNA-Sequenzen umfassen, oder anlagernden Äquivalenten davon;
- Amplifizieren eines Abschnitts originaler DNA mittels PCR unter Verwendung des Primer-Paares, um aus der Probe ein PCR-Produkt oder mehrere PCR-Produkte zu erzeugen und um den besagten einen Standard oder die besagten mehreren Standards bekannter Identität zu erzeugen;
- Vergleichen des besagten einen, aus der Probe gewonnenen PCR-Produktes oder der besagten mehreren, aus der Probe gewonnenen PCR-Produkte mit einem oder mehreren PCR-Produkten des besagten einen Standards oder der besagten mehreren Standards bekannter Identität; und
- Identifizieren der in der Probe vorliegenden Eimeria-Spezies.

20. Verfahren nach Anspruch 19, wobei das PCR-Primer-Paar Primer umfasst, welche die als SEQ ID NO: 25 und SEQ ID NO: 26 bezeichneten Sequenzen umfassen.

21. Verfahren nach Anspruch 19, wobei das PCR-Primer-Paar eine der Sequenzen SEQ ID NO: 9, SEQ ID NO: 11, SEQ ID NO: 13 oder SEQ ID NO: 15, und eine der Sequenzen SEQ ID NO: 10, SEQ ID NO: 12, SEQ ID NO: 14 oder SEQ ID NO: 16 umfasst.

22. Verfahren nach Anspruch 19, wobei das PCR-Primer-Paar die Primer umfasst, welche die als SEQ ID NO: 27 und SEQ ID NO: 28 bezeichneten Sequenzen umfasst.

23. Verfahren nach Anspruch 19, wobei das PCR-Primer-Paar eine der Sequenzen SEQ ID NO: 17, SEQ ID NO: 19, SEQ ID NO: 21 oder SEQ ID NO: 23, und eine der Sequenzen SEQ ID NO: 18, SEQ ID NO: 20, SEQ ID NO: 22 oder SEQ ID NO: 24 umfasst.

24. Verfahren nach einem der vorhergehenden Ansprüche 19 bis 23, wobei pro zu testender Probe zwei PCR durchgeführt werden und wobei für jede PCR jeweils ein unterschiedliches Primer-Paar verwendet wird.

25. Verfahren nach einem der Ansprüche 19 bis 23, wobei pro zu testender Probe eine PCR durchgeführt wird und beide Primer-Paare innerhalb dieser einen PCR bereitgestellt werden.

26. Verfahren nach einem der Ansprüche 19 bis 25, wobei das besagte eine, aus der Probe gewonnene PCR-Produkt oder die besagten mehreren, aus der Probe gewonnenen PCR-Produkte mit dem besagten einen PCR-Produkt oder den besagten mehreren PCR-Produkten des besagten einen Standards oder der besagten mehreren Standards bekannter Identität mittels Gel-Elektrophorese verglichen werden.

27. Verfahren nach Anspruch 26, wobei es sich bei der Gel-Elektrophorese um DPGE handelt.

28. Verfahren nach Anspruch 26, wobei es sich bei der Gel-Elektrophorese um SSCP handelt.

29. Verfahren nach Anspruch 26, wobei sowohl DPGE als auch SSCP eingesetzt werden.

30. Verfahren nach einem der Ansprüche 26 bis 29, wobei die Gel-Elektrophorese in einem automatisierten System durchgeführt wird.

31. Verwendung eines der in den Ansprüchen 1 bis 12 definierten Oligonucleotide, oder eines der anlagernden Äquivalente davon, zur Identifizierung von *Eimeria*-Spezies.

## Revendications

1. Oligonucléotide comprenant au moins 15 bases consécutives de la séquence d'ADN désignée par SEQ ID N° : 25, ou un équivalent d'annelage de celui-ci, dans lequel ledit oligonucléotide est capable d'hybridation spécifique sous des conditions de PCR à une séquence cible qui est complémentaire desdites au moins 15 bases consécutives, et ledit oligonucléotide est adapté pour amorcer l'extension de l'ADN durant la PCR.

2. Oligonucléotide tel que revendiqué selon la revendication 1, comprenant la séquence désignée par SEQ ID N° : 25.

3. Séquence oligonucléotidique telle que revendiquée selon la revendication 1, dans laquelle l'oligonucléotide est choisi parmi le groupe constitué de : SEQ ID N° : 9 ; SEQ ID N° : 11 ; SEQ ID N° : 13 ; et SEQ ID N° : 15.

4. Oligonucléotide comprenant au moins 15 bases consécutives de la séquence d'ADN désignée par SEQ ID N° : 26, ou un équivalent d'annelage de celui-ci, dans lequel ledit oligonucléotide est capable d'hybridation spécifique sous des conditions de PCR à une séquence cible qui est complémentaire desdites au moins 15 bases consécutives, et ledit oligonucléotide est adapté pour amorcer l'extension de l'ADN durant la PCR.

5. Oligonucléotide tel que revendiqué selon la revendication 4, comprenant la séquence désignée par SEQ ID N° : 26.

6. Oligonucléotide tel que revendiqué selon la revendication 4, dans lequel l'oligonucléotide est choisi parmi le groupe constitué de SEQ ID N° : 10 ; SEQ ID N° : 12; SEQ ID N°:14;et SEQ ID N°:16.

7. Oligonucléotide comprenant au moins 15 bases de la séquence d'ADN désignée par SEQ ID N° : 27, ou un équivalent d'annelage de celui-ci, dans lequel ledit oligonucléotide est capable d'hybridation spécifique sous des conditions de PCR à une séquence cible qui est complémentaire desdites au moins 15 bases consécutives, et ledit oligonucléotide est adapté pour amorcer l'extension de l'ADN durant la PCR.

8. Oligonucléotide tel que revendiqué selon la revendication 7, comprenant la séquence désignée par SEQ ID N° : 27.

9. Oligonucléotide tel que revendiqué selon la revendication 7, dans lequel l'oligonucléotide est choisi parmi le groupe constitué de SEQ ID N° : 17 ; SEQ ID N° : 19 ; SEQ ID N° : 21 ; et SEQ ID N° : 23.

10. Oligonucléotide comprenant au moins 15 bases consécutives de la séquence d'ADN désignée par SEQ ID N° : 28, ou un équivalent d'annelage de celui-ci, dans lequel ledit oligonucléotide est capable d'hybridation spécifique sous des conditions de PCR à une séquence cible qui est complémentaire desdites au moins 15 bases consécutives, et ledit oligonucléotide est adapté pour amorcer l'extension de l'ADN durant la PCR.

11. Oligonucléotide tel que revendiqué selon la revendication 10, comprenant une séquence désignée par SEQ ID N° : 28.

12. Oligonucléotide tel que revendiqué selon la revendication 10, dans lequel l'oligonucléotide est choisi parmi le groupe constitué de SEQ ID N° : 18 ; SEQ ID N° : 20 ; SEQ ID N° : 22 ; et SEQ ID N° : 24.

13. Paire d'amorces de PCR, une amorce comprenant au moins 15 bases consécutives de la séquence d'ADN désignée par SEQ ID N° : 25, ou un équivalent d'annelage de celle-ci, et une seconde amorce comprenant au moins 15 bases consécutives de la séquence d'ADN désignée par SEQ ID N° : 26, ou un équivalent d'annelage de celle-ci.

14. Paire d'amorces de PCR telle que revendiquée selon la revendication 13, dans laquelle ladite paire d'amorces de PCR comprend une amorce comprenant une séquence d'ADN désignée par SEQ ID N° : 25 et une seconde amorce comprenant la séquence d'ADN désignée par SEQ ID N° : 26.

15. Paire d'amorces de PCR telle que revendiquée selon la revendication 13, dans laquelle ladite paire d'amorces de PCR comprend une séquence parmi SEQ ID N° : 9; SEQ ID N° : 11 ; SEQ ID N° : 13 ; ou SEQ ID N° :15 ; et une séquence parmi SEQ ID N° : 10 ; SEQ ID N° : 12 ; SEQ ID N° : 14 ; ou SEQ ID N° : 16.

16. Paire d'amorces de PCR, une amorce comprenant au moins 15 bases consécutives de la séquence d'ADN désignée par SEQ ID N° : 27, ou un équivalent d'annelage de celle-ci et une seconde amorce comprenant au moins 15 bases consécutives de la séquence d'ADN désignée par SEQ ID N° : 28, ou un équivalent d'annelage de celle-ci.

17. Paire d'amorces de PCR telle que revendiquée selon la revendication 16, dans laquelle ladite paire d'amorces de PCR comprend une amorce comprenant la séquence d'ADN désignée par SEQ ID N° : 27 et une amorce comprenant la séquence d'ADN désignée par SEQ ID N° : 28.

18. Paire d'amorces de PCR telle que revendiquée selon la revendication 16, dans laquelle ladite paire d'amorces de PCR comprend une séquence parmi SEQ ID N° : 17; SEQ ID N° : 19 ; SEQ ID N° : 21 ; ou SEQ ID N° : 23; et une séquence parmi SEQ ID N° : 18 ; SEQ ID N° : 20 ; SEQ ID N° : 22 ; ou SEQ ID N° : 24.

19. Procédé d'identification *d'Eimeria* dans un échantillon, ledit procédé comprenant les étapes :
- de fourniture d'un échantillon comprenant l'ADN modèle génomique à tester ;
- de fourniture de l'ADN génomique d'un ou plusieurs standard(s) d'identité connue ;
- de fourniture d'une paire d'amorces de PCR choisies parmi le groupe constitué d'
(i) amorces comprenant au moins 15 bases consécutives des séquences d'ADN désignées par SEQ ID N° : 25 et SEQ ID N° : 26 ou les équivalents d'annelage de celles-ci ; ou
(ii) amorces comprenant au moins 15 bases consécutives des séquences d'ADN désignées par SEQ ID N° : 27 et SEQ ID N° : 28 ou les équivalents d'annelage de celles-ci ;
- d'amplification au moyen de la PCR d'une région de l'ADN modèle en utilisant ladite paire d'amorces pour produire un ou plusieurs produit(s) de PCR à partir dudit échantillon, et dudit ou desdits plusieurs standard(s) d'identité connue ;
- de comparaison dudit ou desdits plusieurs produit(s) de PCR dérivé(s) dudit échantillon par rapport à un ou plusieurs produit(s) de PCR dudit un ou desdits plusieurs standard(s) d'identité connue ; et
- d'identification des espèces *d'Eimeria* présentes dans l'échantillon.

20. Procédé tel que revendiqué selon la revendication 19, dans lequel ladite paire d'amorces de PCR comprend les amorces comprenant des séquences désignées par SEQ ID N° : 25 et SEQ ID N° : 26.

21. Procédé tel que revendiqué selon la revendication 19, dans lequel ladite paire d'amorces de PCR comprend une séquence parmi SEQ ID N° : 9 ; SEQ ID N° : 11 ; SEQ ID N° : 13; ou SEQ ID N° : 15 et une séquence parmi SEQ ID N° : 10; SEQ ID N° : 12, SEQ ID N° : 14 ; ou SEQ ID N° : 16.

22. Procédé tel que revendiqué selon la revendication 19, dans lequel ladite paire d'amorces de PCR comprend les amorces comprenant des séquences désignées par SEQ ID N° : 27 et SEQ ID N° : 28.

23. Procédé tel que revendiqué selon la revendication 19, dans lequel ladite paire d'amorces comprend une séquence parmi SEQ ID N° : 17; SEQ ID N° : 19; SEQ ID N° : 21 ; ou SEQ ID N° : 23 et une séquence parmi SEQ ID N° : 18 ; SEQ ID N° : 20 ; SEQ ID N° : 22 ; ou SEQ ID N° : 24.

24. Procédé tel que revendiqué selon l'une quelconque des revendications 19 à 23, dans lequel deux PCR sont conduites par échantillon à tester, et dans lequel chaque PCR utilise une paire différente d'amorces.

25. Procédé tel que revendiqué selon l'une quelconque des revendications 19 à 23, dans lequel une PCR est conduite par échantillon à tester et les deux paires d'amorces sont fournies à l'intérieur de ladite une PCR.

26. Procédé tel que revendiqué selon l'une quelconque des revendications 19 à 25, dans lequel ledit ou lesdits plusieurs produit(s) de PCR dérivés dudit échantillon sont comparés au dit ou aux dits un ou plusieurs produit(s) de PCR dudit un ou desdits plusieurs standard(s) d'identité connue au moyen d'une électrophorèse sur gel.

27. Procédé tel que revendiqué selon la revendication 26, dans lequel l'électrophorèse sur gel est la DPGE.

28. Procédé tel que revendiqué selon la revendication 26, dans lequel l'électrophorèse sur gel est la détection du SSCP.

29. Procédé tel que revendiqué selon la revendication 26, dans lequel à la fois la DPGE et la détection du SSCP sont utilisées.

30. Procédé tel que revendiqué selon l'une quelconque des revendications 26 et 29, dans lequel l'électrophorèse sur gel est conduite dans un système automatisé.

31. Utilisation de l'un quelconque des oligonucléotides définis selon les revendications 1 à 12, ou des équivalents d'annelage de ceux-ci, pour l'identification d'espèces *d'Eimeria.*
